(19) **Europäisches Patentamt / European Patent Office / Office européen des brevets**

(11) **EP 4 772 624 A1**

(12) **EUROPEAN PATENT APPLICATION**
published in accordance with Art. 153(4) EPC

(43) Date of publication:
08.07.2026 Bulletin 2026/28

(21) Application number: 24859877.3

(22) Date of filing: 29.08.2024

(51) International Patent Classification (IPC):
C12N 9/52 (2006.01)   C07K 16/18 (2006.01)
C07K 19/00 (2006.01)   C12N 1/15 (2006.01)
C12N 1/19 (2006.01)   C12N 1/21 (2006.01)
C12N 5/10 (2006.01)   C12N 9/10 (2006.01)
C12N 15/62 (2006.01)   C12N 15/63 (2006.01)

(52) Cooperative Patent Classification (CPC):
C07K 16/18; C07K 19/00; C12N 5/10; C12N 9/10;
C12N 9/52; C12N 15/62; C12N 15/63; C12N 15/74;
C12N 15/80; C12N 15/81

(86) International application number:
PCT/JP2024/030890

(87) International publication number:
WO 2025/047848 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.08.2023 JP 2023139803

(71) Applicant: Astellas Pharma Inc.
5-1, Nihonbashi-honcho 2-chome,
Chuo-ku
Tokyo 103-8411 (JP)

(72) Inventors:
• IWASAKI, Masashi
Tokyo, 1038411 (JP)

• YAMAJUKU, Daisuke
Tokyo, 1038411 (JP)
• YAMAZAKI, Rika
Tokyo, 1038411 (JP)
• TOMIMOTO, Yusuke
Tokyo, 1038411 (JP)
• SHIRAI, Hiroki
Tokyo, 1038411 (JP)

(74) Representative: Hoffmann Eitle
Patent- und Rechtsanwälte PartmbB
Arabellastraße 30
81925 München (DE)

Remarks:
The complete document including Reference Table(s) and the Sequence Listing(s) can be downloaded from the EPO website

(54) **IdeZ VARIANT**

(57) According to one embodiment, provided is an IdeZ variant having IgG cysteine protease activity, the variant (a) comprising a sequence 90% or more identical to an amino acid sequence from positions 35 to 349 of the amino acid sequence represented by SEQ ID NO: 1; and (b) having unglycosylated amino acids at a position corresponding to position 118 and a position corresponding to position 267 of the amino acid sequence represented by SEQ ID NO: 1.

EP 4 772 624 A1

**Description**

Technical Field

The present invention relates to an IdeZ variant and a polypeptide and the like comprising the IdeZ variant.

Background Art

IdeS derived from *Streptococcus* is a cysteine protease that sequence-specifically cleaves a hinge connecting Fab and Fc of human IgG. This enzyme has high cleavage efficiency and substrate specificity and as such, is utilized in the characterization of antibody drug candidates, and the like. In recent years, its use has been expanded, and, for example, use in high-quality recombinant protein production systems and effects on rejection in transplantation, autoimmune diseases, and the like have been expected (Non Patent Literatures 1 and 2). Furthermore, fusion products of IdeS and a human Fc region are also under research (Non Patent Literature 1).

However, the high antigenicity of IdeS may become problematic in such a way that humans having a history of *Streptococcus* infection have immunity against IdeS and such humans produce an anti-drug antibody (ADA) upon administration of IdeS (Non Patent Literature 3). In order to solve such problems with IdeS, research is also underway on IdeZ derived from a *Streptococcus* that does not infect humans, and on IdeZ variants having a variations that reduce ADA production, and the like (Patent Literatures 1 and 2). There is a demand for further improvement in characteristics of such IdeZ or IdeZ variants.

Citation List

Patent Literature

Patent Literature 1: International Publication No. WO 2020/159970
Patent Literature 2: International Publication No. WO 2021/233911

Non Patent Literature

Non Patent Literature 1: The 1st Academic Conference in Commemoration of the Establishment of the Antibody Society of Japan, Nov. 2022, P-74
Non Patent Literature 2: Drugs, Oct. 2020, Vol. 80, p. 1859-1864
Non Patent Literature 3: Am J Transplant, Nov. 2018, Vol. 18 (11), p. 2752-2762

Summary of Invention

Technical Problem

The present inventors have conducted diligent studies to obtain IgG cysteine protease having much better characteristics and in the course thereof, have found that a problem in the production of wild-type IdeZ using mammalian cells (e.g., CHO cells) is the attenuation of protease activity. An object of the present invention is to provide an IdeZ variant that has favorable protease activity even when produced using mammalian cells, and a polypeptide and the like comprising the IdeZ variant.

Solution to Problem

To address the object as mentioned above, the present inventors have further conducted diligent studies and consequently found that favorable protease activity can be obtained by an amino acid variation at a specific position (e.g., the consensus sequence of an N-linked sugar chain) of IdeZ, even when the IdeZ is produced using mammalian cells.

The present invention is, for example, as follows.

[1] An IdeZ variant having IgG cysteine protease activity, the variant

(a) comprising a sequence 90% or more identical to an amino acid sequence from positions 35 to 349 of the amino acid sequence represented by SEQ ID NO: 1; and

(b) having unglycosylated amino acids at a position corresponding to position 118 and a position corresponding to position 267 of the amino acid sequence represented by SEQ ID NO: 1.

[2] The IdeZ variant according to [1], wherein the IdeZ variant comprises the following amino acid variations (b') and (b"):

(b')

(i) an amino acid at position corresponding to position 118 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;

(ii) an amino acid at position corresponding to position 119 of the amino acid sequence represented by SEQ ID NO: 1 is substituted by proline; and/or

(iii) an amino acid at position corresponding to position 120 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane; and

(b")

(i) an amino acid at position corresponding to position 267 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;

(ii) an amino acid at position corresponding to position 268 of the amino acid sequence represented by SEQ ID NO: 1 is substituted by proline; and/or

(iii) an amino acid at position corresponding to position 269 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane.

[3] The IdeZ variant according to [2], wherein in the IdeZ variant, an amino acid at position corresponding to position 120 of the amino acid sequence represented by SEQ ID NO: 1 is substituted by glutamic acid, and an amino acid at position corresponding to position 269 of the amino acid sequence represented by SEQ ID NO: 1 is substituted by arginine.

[4] The IdeZ variant according to any of [1] to [3], wherein the IdeZ variant has (c) an unglycosylated amino acid at position corresponding to position 171 of the amino acid sequence represented by SEQ ID NO: 1, and/or (d) an unglycosylated amino acid at position corresponding to position 298 of the amino acid sequence represented by SEQ ID NO: 1.

[5] The IdeZ variant according to [4], wherein the IdeZ variant comprises the following amino acid variations (c') and/or (d'):

(c')

(i) an amino acid at position corresponding to position 171 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;

(ii) an amino acid at position corresponding to position 172 of the amino acid sequence represented by SEQ ID NO: 1 is substituted by proline; and/or

(iii) an amino acid at position corresponding to position 173 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane;

(d')

(i) an amino acid at position corresponding to position 298 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;
(ii) an amino acid at position corresponding to position 299 of the amino acid sequence represented by SEQ ID NO: 1 is substituted by proline; and/or
(iii) an amino acid at position corresponding to position 300 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane.

[6] The IdeZ variant according to [5], wherein in the IdeZ variant, an amino acid at position corresponding to position 171 of the amino acid sequence represented by SEQ ID NO: 1 is substituted by glycine, and/or an amino acid at position corresponding to position 298 of the amino acid sequence represented by SEQ ID NO: 1 is substituted by aspartic acid.

[7] The IdeZ variant according to [6], wherein

in the IdeZ variant,
an amino acid at position corresponding to position 120 of the amino acid sequence represented by SEQ ID NO: 1 is substituted by glutamic acid,
an amino acid at position corresponding to position 171 of the amino acid sequence represented by SEQ ID NO: 1 is substituted by glycine,
an amino acid at position corresponding to position 269 of the amino acid sequence represented by SEQ ID NO: 1 is substituted by arginine, and
an amino acid at position corresponding to position 298 of the amino acid sequence represented by SEQ ID NO: 1 is substituted by aspartic acid.

[8] The IdeZ variant according to [7], wherein the IdeZ variant comprises an amino acid sequence 90% or more identical to the amino acid sequence represented by SEQ ID NO: 5.

[9] A polypeptide comprising the IdeZ variant according to any of [1] to [8].

[10] The polypeptide according to [9], wherein the polypeptide is a fusion protein of the IdeZ variant and an Fc region.

[11] The polypeptide according to [10], wherein the Fc region is derived from human IgG.

[12] The polypeptide according to [11], wherein the Fc region is derived from human Igγ1, human Igy2, human Igy3, or human Igy4.

[13] The polypeptide according to any of [10] to [12], wherein

in the Fc region,
amino acid residue 236 is substituted by glutamic acid, and
amino acid residue 237 is substituted by valine,

wherein the amino acid residue numbers are amino acid positions according to the EU index.

[14] The polypeptide according to [13], wherein

in the Fc region, in addition,
amino acid residue 234 is substituted by alanine, and
amino acid residue 235 is substituted by glutamic acid.

[15] The polypeptide according to [14], wherein

in the Fc region, in addition,
amino acid residue 227 is substituted by histidine,
amino acid residue 228 is substituted by lysine,
amino acid residue 233 is substituted by asparagine, and/or
amino acid residue 238 is substituted by serine.

[16] The polypeptide according to [14] or [15], wherein

in the Fc region, in addition,
amino acid residue 253 is substituted by alanine,
amino acid residue 293 or 294 is deleted, and/or
amino acid residue 296 is substituted by alanine.

[17] The polypeptide according to [16], wherein the Fc region is selected from the following Fc regions (1) and (2):

(1)

an Fc region in which
amino acid residue 234 is substituted by alanine,
amino acid residue 235 is substituted by glutamic acid,
amino acid residue 236 is substituted by glutamic acid,
amino acid residue 237 is substituted by valine,
amino acid residue 253 is substituted by alanine, and
amino acid residue 293 or 294 is deleted; and

(2)

an Fc region in which
amino acid residue 227 is substituted by histidine,
amino acid residue 228 is substituted by lysine,
amino acid residue 233 is substituted by asparagine,
amino acid residue 234 is substituted by alanine,
amino acid residue 235 is substituted by glutamic acid,
amino acid residue 236 is substituted by glutamic acid,
amino acid residue 237 is substituted by valine,
amino acid residue 238 is substituted by serine,
amino acid residue 253 is substituted by alanine, and
amino acid residue 293 or 294 is deleted.

[18] The polypeptide according to [17], wherein the Fc region comprises an amino acid sequence 90% or more identical to a sequence consisting of amino acids at positions 316 to 541 in the amino acid sequence represented by SEQ ID NO: 16, a sequence consisting of amino acids at positions 316 to 541 in the amino acid sequence represented by SEQ ID NO: 17, a sequence consisting of amino acids at positions 316 to 541 in the amino acid sequence represented by SEQ ID NO: 21, or a sequence consisting of amino acids at positions 316 to 541 in the amino acid sequence represented by SEQ ID NO: 22.
[19] The polypeptide according to any of [10] to [18], wherein the fusion protein is a fusion protein of the IdeZ variant linked to the N terminus of the Fc region, and two such fusion proteins form a dimer.
[20] The polypeptide according to any of [10] to [18], wherein the polypeptide comprises a fusion protein of the IdeZ variant linked to the N terminus of a first Fc region, and a second Fc region without the IdeZ variant linked thereto.
[21] The polypeptide according to [20], wherein

the first Fc region and the second Fc region comprise knobs-into-holes mutations, and
the knobs-into-holes mutations are any of the following mutations (1) and (2):

(1)

in the first Fc region, amino acid residue 366 is substituted by tryptophane, and
in the second Fc region, amino acid residue 366 is substituted by serine, amino acid residue 368 is substituted by alanine, and amino acid residue 407 is substituted by valine; and

(2)

in the first Fc region, amino acid residue 366 is substituted by serine, amino acid residue 368 is substituted by alanine, and amino acid residue 407 is substituted by valine, and
in the second Fc region, amino acid residue 366 is substituted by tryptophane.

[22] The polypeptide according to any of [19] to [21], wherein the Fc region or the first Fc region and the second Fc region are sialylated.

[23] A polynucleotide encoding the polypeptide according to any of [9] to [22].

[24] An expression vector comprising the polynucleotide according to [23].

[25] A host cell transformed with the expression vector according to [24].

[26] A method for producing a polypeptide comprising an IdeZ variant, the method comprising the step of culturing the host cell according to [25].

[27] The method according to [26], further comprising the step of adding or expressing β-galactoside α-2,6-sialyltransferase 1 (ST6GAL1).

[28] An Fc region variant wherein

amino acid residue 236 is substituted by glutamic acid, and
amino acid residue 237 is substituted by valine,

wherein the amino acid residue numbers are amino acid positions according to the EU index.

[29] The Fc region variant according to [28], wherein, in addition,

amino acid residue 234 is substituted by alanine, and
amino acid residue 235 is substituted by glutamic acid.

[30] The Fc region variant according to [29], further comprising the following amino acid variation(s):

amino acid residue 227 is substituted by histidine,
amino acid residue 228 is substituted by lysine,
amino acid residue 233 is substituted by asparagine, and/or
amino acid residue 238 is substituted by serine.

[31] The Fc region variant according to [29] or [30], further comprising the following amino acid variation(s):

amino acid residue 253 is substituted by alanine,
amino acid residue 293 or 294 is deleted, and/or
amino acid residue 296 is substituted by alanine.

[32] The Fc region variant according to [31], wherein the Fc region variant is selected from the following Fc region variants (1) and (2):

(1)

an Fc region variant in which
amino acid residue 234 is substituted by alanine,
amino acid residue 235 is substituted by glutamic acid,
amino acid residue 236 is substituted by glutamic acid,
amino acid residue 237 is substituted by valine,
amino acid residue 253 is substituted by alanine, and
amino acid residue 293 or 294 is deleted; and

(2)

an Fc region variant in which
amino acid residue 227 is substituted by histidine,
amino acid residue 228 is substituted by lysine,
amino acid residue 233 is substituted by asparagine,
amino acid residue 234 is substituted by alanine,
amino acid residue 235 is substituted by glutamic acid,
amino acid residue 236 is substituted by glutamic acid,
amino acid residue 237 is substituted by valine,
amino acid residue 238 is substituted by serine,
amino acid residue 253 is substituted by alanine, and

amino acid residue 293 or 294 is deleted.

[33] The Fc region variant according to any of [28] to [32], wherein the Fc region is derived from human IgG.

[34] The Fc region variant according to [33], wherein the Fc region is derived from human Igγ1, human Igy2, human Igy3, or human Igy4.

[35] A polypeptide comprising the Fc region variant according to any of [28] to [34] which is not cleaved with IgG cysteine protease.

[36] A fusion protein of IgG cysteine protease and the Fc region variant according to any of [28] to [34].

[37] The fusion protein according to [36], wherein the IgG cysteine protease is IdeS, IdeZ, IdeZ2, IdeE, IdeE2, IdeP, IdeSORK, or IgdE, or a variant thereof.

[38] The fusion protein according to [37], wherein the fusion protein comprises an amino acid sequence 90% or more identical to a sequence consisting of amino acids at positions 316 to 541 in the amino acid sequence represented by SEQ ID NO: 16, a sequence consisting of amino acids at positions 316 to 541 in the amino acid sequence represented by SEQ ID NO: 17, a sequence consisting of amino acids at positions 316 to 541 in the amino acid sequence represented by SEQ ID NO: 21, or a sequence consisting of amino acids at positions 316 to 541 in the amino acid sequence represented by SEQ ID NO: 22.

Advantageous Effects of Invention

The present invention can provide an IdeZ variant that has favorable protease activity even when produced using mammalian cells, and a polypeptide and the like comprising the IdeZ variant.

Brief Description of Drawings

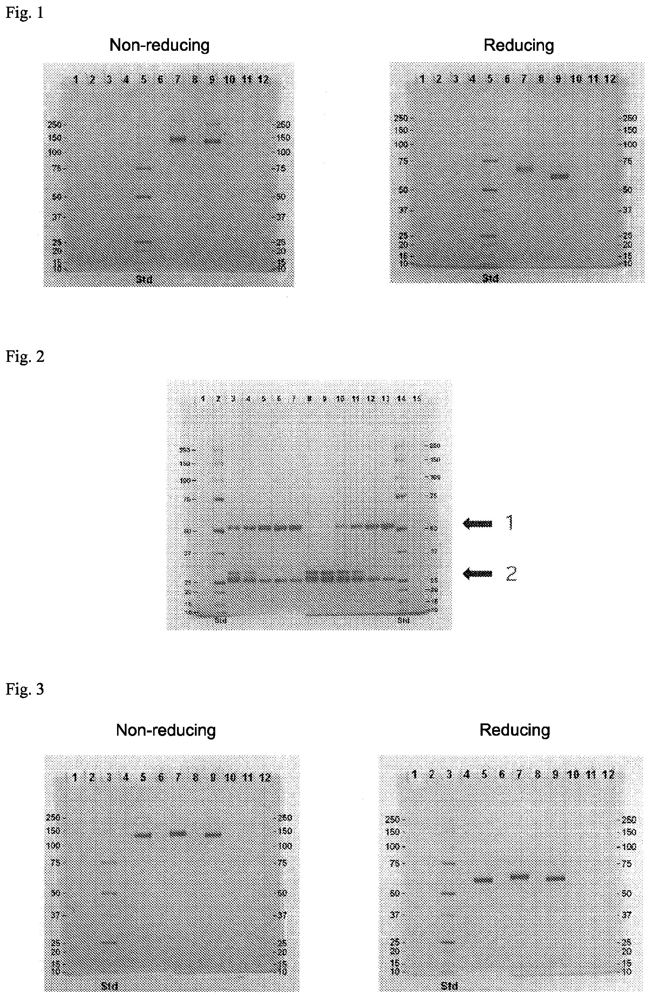

[Figure 1] Figure 1 shows the electrophoretic profile of each fusion protein produced in Example 1 (left diagram: a non-reducing condition, right diagram: a reducing condition). Lane 5 depicts a molecular weight marker, lane 7 depicts a wild-type IdeZ-mouse Fc fusion protein, and lane 9 depicts an IdeZ variant A-mouse Fc fusion protein.

[Figure 2] Figure 2 shows the cleaving activity of each fusion protein produced in Example 1. Arrow 1 depicts an H chain. Arrow 2 depicts a Fab' fragment. Lanes 3 to 7 depict the electrophoretic profiles of an antibody cleaved with the wild-type IdeZ-mouse Fc fusion protein at antibody concentration ratios of 10, 33, 100, 333, and 1000 times, respectively. Lanes 8 to 12 depict the electrophoretic profiles of an antibody tested in the same manner as above using the IdeZ variant A-mouse Fc fusion protein. Lane 13 depicts the electrophoretic profile of only anti-KLH_hIgG1.

[Figure 3] Figure 3 shows the electrophoretic profile of each fusion protein produced in Example 2 (left diagram: a non-reducing condition, right diagram: a reducing condition). Lane 5 depicts an IdeZ variant 2-mouse Fc fusion protein, lane 7 depicts an IdeZ variant 3-mouse Fc fusion protein, and lane 9 depicts an IdeZ variant 4-mouse Fc fusion protein.

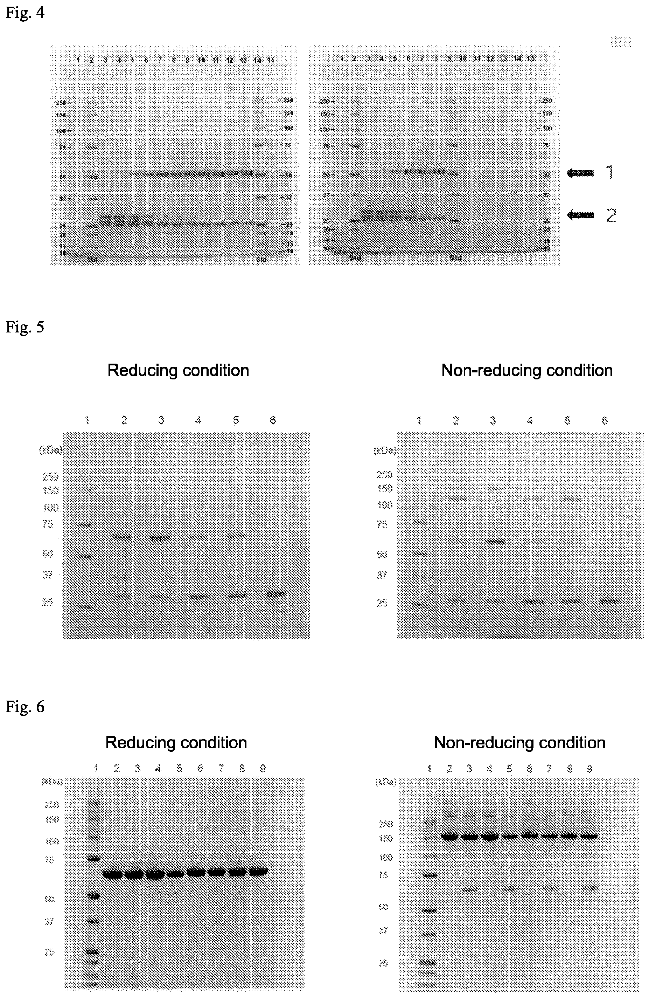

[Figure 4] Figure 4 shows the cleaving activity of each fusion protein produced in Example 2. Arrow 1 depicts an H chain. Arrow 2 depicts a Fab' fragment. Lanes 3 to 7 of the left diagram depict the electrophoretic profiles of an antibody cleaved with the IdeZ variant 2-mouse Fc fusion protein at antibody concentration ratios of 10, 33, 100, 333, and 1000 times, respectively. Lanes 8 to 12 depict the electrophoretic profiles of an antibody tested in the same manner as above using the IdeZ variant 3-mouse Fc fusion protein. Lanes 3 to 7 of the right diagram depict the electrophoretic profiles of an antibody tested in the same manner as above using the IdeZ variant 4-mouse Fc fusion protein.

[Figure 5] Figure 5 shows the electrophoretic profile of each fusion protein produced in Example 3-1 (left diagram: a reducing condition, right diagram: a non-reducing condition). Lane 1 depicts a molecular weight marker, and lanes 2 to 6 depict sialylated IdeZ variant A-human Fc variant 5, sialylated IdeZ variant A-human Fc variant 6, sialylated IdeZ variant A-human Fc variant 9, sialylated IdeZ variant A-human Fc variant 10, and sialylated IdeZ variant A-human Fc variant 11, respectively.

[Figure 6] Figure 6 shows the electrophoretic profile of each fusion protein produced in Example 3-2 (left diagram: a reducing condition, right diagram: a non-reducing condition). Lane 1 depicts a molecular weight marker, and lanes 2 to 5 depict non-sialylated IdeZ variant A-human Fc variants 7, 8, 17, and 18, respectively. Lanes 6 to 9 depict sialylated IdeZ variant A-human Fc variants 7, 8, 17, and 18, respectively.

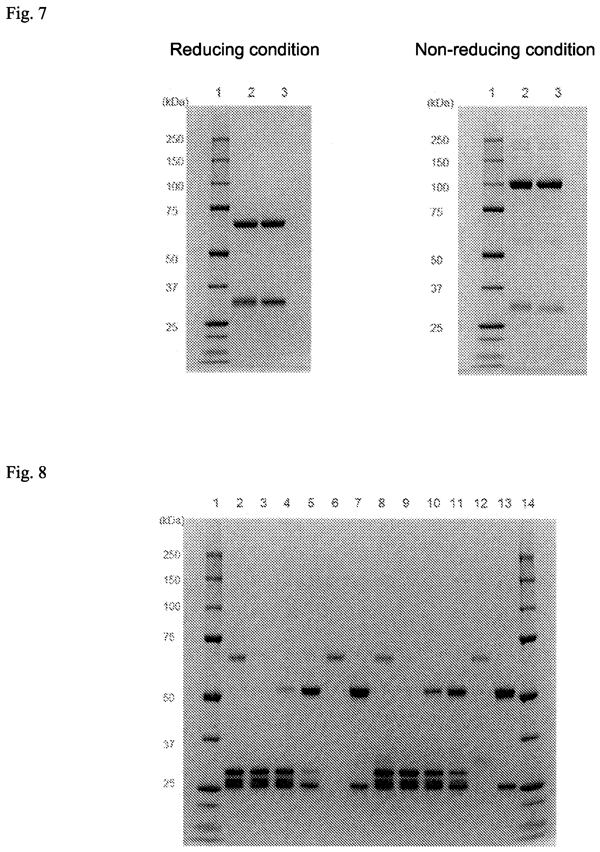

[Figure 7] Figure 7 shows the electrophoretic profile of each fusion protein produced in Example 4 (left diagram: a reducing condition, right diagram: a non-reducing condition). Lane 1 depicts a molecular weight marker, lane 2 depicts non-sialylated IdeZ variant A-human Fc variant 19, and lane 3 depicts sialylated IdeZ variant A-human Fc variant 19.

[Figure 8] Figure 8 shows the cleaving activity of each fusion protein produced in Example 5. Lanes 2 to 5 depict electrophoretic profiles after sialylated IdeZ variant A-human Fc variant 7 was reacted at final concentrations of 0.2,

EP 4 772 624 A1

0.04, 0.008, and 0.0016 times, respectively, the amount of anti-KLH_hIgGI. Lanes 8 to 11 depict electrophoretic profiles after sialylated IdeZ variant A-human Fc variant 19 was reacted at final concentrations of 0.2, 0.04, 0.008, and 0.0016 times, respectively, the amount of anti-KLH_hIgG1. Lanes 1 and 14 each depict a molecular weight marker, lanes 7 and 13 each depict the electrophoretic profile of anti-KLH_hIgG1, lane 6 depicts the electrophoretic profile of the sialylated IdeZ variant A-human Fc variant 7, lane 12 depicts the electrophoretic profile of the sialylated IdeZ variant A-human Fc variant 19.

Description of Embodiments

Hereinafter, the mode for carrying out the present invention will be described in detail. Materials, configurations, and the like described below do not limit the present invention, and various changes or modifications can be made in the present invention without departing from the spirit of the present invention.

1. Definition

Terms in the present specification are used in meanings generally used by those skilled in the art, unless otherwise specified.

As used herein, the term "IdeZ variant" means IdeZ comprising at least one amino acid variation in wild-type IdeZ (SEQ ID NO: 1), and the amino acid variation includes amino acid substitution, addition, deletion, insertion, modification, and the like. As used herein, the "variation" and the "mutation" of an amino acid are used interchangeably with each other.

As used herein, the term "Fc region variant" means an Fc region comprising at least one amino acid variation in a wild-type Fc region, and the amino acid variation includes amino acid substitution, addition, deletion, insertion, modification, and the like. As used herein, the term "Fc region" or "Fc region variant" used is meant to also include a complex (e.g., a dimer) composed of a plurality of Fc regions.

As used herein, the "Fc region (variant)" may be derived from any antibody. The antibody (or immunoglobulin) refers to a glycoprotein having a four-chain structure having a bilaterally symmetrical Y-shaped structure composed of two heavy chains having a single sequence and two light chains having a single sequence. The antibody has five classes: IgG, IgM, IgA, IgD, and IgE. The basic structure of the antibody molecule is common among these classes, and two heavy chains having a molecular weight of 50,000 to 70,000 and two light chains having a molecular weight of 20,000 to 30,000 are bound through a disulfide bond and a noncovalent bond to form an antibody molecule having a Y-shaped four-chain structure with a molecular weight of 150,000 to 190,000.

The heavy chain consists of a polypeptide chain usually containing approximately 440 amino acids, has a structure characteristic of each class, and is designated as Ig$\gamma$, Ig$\mu$, Ig$\alpha$, Ig$\delta$, and Ig$\epsilon$ correspondingly to IgG, IgM, IgA, IgD, and IgE, respectively. IgG further has subclasses IgG1, IgG2, IgG3, and IgG4, and heavy chains corresponding thereto are designated as Ig$\gamma$1, Ig$\gamma$2, Ig$\gamma$3, and Ig$\gamma$4, respectively. The light chain consists of a polypeptide chain usually containing approximately 220 amino acids, and is known to have two types, L type and K type, which are designated as Ig$\lambda$ and Ig$\kappa$, respectively. Each of these two types of light chains can be paired with any type of heavy chain.

In an antibody molecule, the heavy chain has four intrachain disulfide bonds (five in Ig$\mu$ and Ig$\epsilon$) while the light chain has two intrachain disulfide bonds, and one loop is formed every 100 to 110 amino acid residues. Their three-dimensional structures are similar among the loops and are designated as structural units or domains. In both the heavy chain and the light chain, a domain positioned at the N terminus is designated as a variable region, which has diverse amino acid sequences even among antibodies produced from the same class (or subclass) of the same species of animals, and is known to participate in antibody-antigen binding specific binding. A domain on the C-terminal side downstream of the variable region on the C-terminal side has an amino acid sequence substantially constant in each class or subclass, and is designated as a constant region. The heavy chain has, in the direction from the N terminus toward the C terminus, a heavy chain variable region (VH) and a heavy chain constant region (CH). CH is further divided, from the N-terminal side, into three domains: a CH1 domain, a CH2 domain, and a CH3 domain (in the case of IgG). The light chain has, in the direction from the N terminus toward the C terminus, a light chain variable region (VL) and a light chain constant region (CL).

The amino acid sequences of three complementarity-determining regions (CDRs) present in VH and VL vary very largely and contribute to the variability of the variable regions. CDRs reside in the order of CDR1, CDR2, and CDR3 at the N terminus of each of the heavy chain and the light chain, and these regions are composed of approximately 5 to 10 amino acid residues and form an antigen binding site. On the other hand, a portion except for CDRs of the variable region is designated as framework regions (FRs), which consist of FR1 to FR4 and comparatively little vary in amino acid sequence.

When an antibody is treated with protease papain, three antibody fragments are obtained. Two N-terminal fragments are designated as Fab (fragment, antigen binding) regions, and the C-terminal fragment is designated as an Fc (fragment, crystallizable) region. As used herein, the "Fc region" means a polypeptide composed of an antibody constant region except for the CH1 domain. Thus, the Fc regions of IgA, IgD, and IgG have C-terminal last two heavy chain constant

8

region domains (CH2 and CH3), and the Fc regions of IgE and IgM comprise C-terminal last three heavy chain constant region domains (CH2, CH3, and CH4). A hinge region, a J chain, and the like, if present, are also included in the term "Fc region". For example, the Fc region of human IgG1 includes a polypeptide composed of the CH2 domain and the CH3 domain, and a polypeptide composed of a portion or the whole of the hinge region, and the CH2 domain and the CH3 domain. Fc regions in antibodies of other classes can be determined by sequence alignment with the human IgG1 heavy chain or a fragment thereof.

As used herein, the term "IdeZ variant-Fc region (or Fc region variant) fusion product" (i.e., fusion protein) is meant to include both of a structure where one IdeZ variant is linked to each of the N termini of a complex of two Fc regions, and a one-armed structure where the IdeZ variant is linked to only one of the Fc regions.

Terms such as "sialylated IdeZ variant-Fc region (or Fc region variant) fusion product" and "sialylated fusion protein" mean a fusion product or the like of an IdeZ variant and an Fc region (or Fc region variant) to which sialic acid is intentionally added by a sialylation step (e.g., treatment with β-galactoside α-2,6-sialyltransferase 1) usually used in the art. On the other hand, as used herein, terms such as "non-sialylated IdeZ variant-Fc region (or Fc region variant) fusion product" and "non-sialylated fusion protein" mean a fusion product or the like of an IdeZ variant and an Fc region (or Fc region variant) without the sialylation step mentioned above, and the fusion protein is not precluded to contain sialic acid.

As used herein, the term "consensus sequence of an N-linked sugar chain" in IdeZ means a sequence positioned at a glycosylation site, which is required for modifying a sugar chain linked to an asparagine residue of a polypeptide, and is, for example, a sequence of asparagine (N)-X-serine (S)/threonine (T) (wherein X is an amino acid other than proline). The wild-type IdeZ represented by SEQ ID NO: 1 has the consensus sequence at positions 118 to 120, positions 171 to 173, positions 267 to 269, and positions 298 to 300.

The amino acid residue numbers of an antibody sequence such as an Fc region used in the present specification are prescribed in accordance with the EU index of Kabat et al. (Kabat et al., Sequences of Proteins of Immunological Interest, 5th Ed, 1991, NIH Publication No. 91-3242).

As used herein, the term "first" or "second" is used for convenient discrimination if two or more types of moieties are present. Use of such a term does not intend to provide a specific order or meaning, unless otherwise specified.

In the present specification, a peptide linker may or may not be present. The "peptide linker" means one or more arbitrary amino acid residues that can be introduced by genetic engineering in order to link two or more moieties. The length of the peptide linker used in the present invention is not particularly limited and can be appropriately selected by those skilled in the art depending on a purpose.

As used herein, the term "identity" means an identity value obtained with parameters provided as defaults using EMBOSS Needle (Nucleic Acids Res., 2015, Vol. 43, p W580-W584). The parameters are as follows.

Gap Open Penalty = 10
Gap Extend Penalty = 0.5
Matrix = EBLOSUM62
End Gap Penalty = false

As used herein, the term "subject" means a human or other animals in need of prevention or treatment. In a certain aspect, the subject is a human in need of prevention or treatment.

2. IdeZ variant

(1) IdeZ variant

The IdeZ variant according to one embodiment of the present invention is an IdeZ variant having IgG cysteine protease activity, the variant

(a) comprising a sequence 90% or more identical to an amino acid sequence from positions 35 to 349 of the amino acid sequence represented by SEQ ID NO: 1 (wild-type IdeZ); and
(b) having unglycosylated amino acids at a position corresponding to position 118 and a position corresponding to position 267 of the amino acid sequence represented by SEQ ID NO: 1.

The present inventors have found that the attenuation of the protease activity of IdeZ having such unglycosylated amino acids at positions 118 and 267 is suppressed even when the IdeZ is expressed in mammalian cells. At the same time, the present inventors have also found that a problem in the production of wild-type IdeZ using mammalian cells is a decreased expression level; and such decrease in expression level is suppressed when the IdeZ variant mentioned above is produced using mammalian cells.

Glycosylation is prevented at the positions mentioned above, for example, by varying the amino acids at the position corresponding to position 118 and the position corresponding to position 267 to amino acids other than asparagine, or adding an amino acid variation to the consensus sequence of an N-linked sugar chain including these positions. The glycosylation is prevented by adding at least one amino acid variation to the aforementioned consensus sequence of the N-linked sugar chain containing asparagine. However, no sugar chain may be attached to a sequence corresponding to the consensus sequence, depending on its amino acid sequence. For example, for a consensus sequence NXS/T, it has been reported that NXT may have higher glycosylation efficiency than that of NXS (when X is the same amino acid and both the sequences are located at the same position in proteins) (Biochem. J., 1981, Vol. 195, p. 639-644; Biochemistry, 1999, Vol. 38, p. 13584-13591; Biochem. J., 2001, Vol. 355, 245-248; and Biochem. J., 1997, Vol. 323, 415-419).

According to one embodiment, the IdeZ variant may comprise amino acid variations (b') and (b") given below. Such amino acid variations can produce an IdeZ variant having unglycosylated amino acids at a position corresponding to position 118 and a position corresponding to position 267 of the amino acid sequence represented by SEQ ID NO: 1. Each of the amino acid variations (b') and (b") can have at least one or more of the variations (i) to (iii):

(b')

(i) an amino acid at position corresponding to position 118 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;
(ii) an amino acid at position corresponding to position 119 of the amino acid sequence represented by SEQ ID NO: 1 is substituted by proline; and/or
(iii) an amino acid at position corresponding to position 120 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane; and

(b")

(i) an amino acid at position corresponding to position 267 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;
(ii) an amino acid at position corresponding to position 268 of the amino acid sequence represented by SEQ ID NO: 1 is substituted by proline; and/or
(iii) an amino acid at position corresponding to position 269 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane.

Preferably, in the IdeZ variant, an amino acid at position corresponding to position 120 of the amino acid sequence represented by SEQ ID NO: 1 is substituted by glutamic acid, and an amino acid at position corresponding to position 269 of the amino acid sequence represented by SEQ ID NO: 1 is substituted by arginine.

The IdeZ variant preferably further has (c) an unglycosylated amino acid at position corresponding to position 171 of the amino acid sequence represented by SEQ ID NO: 1, and/or (d) an unglycosylated amino acid at position corresponding to position 298 of the amino acid sequence represented by SEQ ID NO: 1. In other words, the unglycosylated amino acids as positions 118 and 267 as mentioned above as well as unglycosylated amino acids at positions 171 and/or 298 are preferred. This is because the protease activity is more improved by decreasing the amount of a sugar chain added. Glycosylation is prevented, for example, by varying the amino acids at the position corresponding to position 171 and/or the position corresponding to position 298 to amino acids other than asparagine, or adding an amino acid variation to the consensus sequence of an N-linked sugar chain including these positions. Specific contents of the amino acid variation that prevents sugar chain attachment are the same as mentioned about the amino acid variation at the position corresponding to position 118 and the position corresponding to position 267 described above.

Such a variation is, for example, as described below. According to one embodiment, the IdeZ variant comprises the following amino acid variations (c') and/or (d'):

(c')

(i) an amino acid at position corresponding to position 171 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;

(ii) an amino acid at position corresponding to position 172 of the amino acid sequence represented by SEQ ID NO: 1 is substituted by proline; and/or

(iii) an amino acid at position corresponding to position 173 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane; and

(d')

(i) an amino acid at position corresponding to position 298 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;

(ii) an amino acid at position corresponding to position 299 of the amino acid sequence represented by SEQ ID NO: 1 is substituted by proline; and/or

(iii) an amino acid at position corresponding to position 300 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane.

Preferably, in the IdeZ variant according to an embodiment, an amino acid at position corresponding to position 171 of the amino acid sequence represented by SEQ ID NO: 1 is substituted by glycine, and/or an amino acid at position corresponding to position 298 of the amino acid sequence represented by SEQ ID NO: 1 is substituted by aspartic acid.

More preferably, the IdeZ variant according to an embodiment has two to four amino acid variations selected from the following amino acid variations (a) to (d):

(a) an amino acid at position corresponding to position 120 of the amino acid sequence represented by SEQ ID NO: 1 is substituted by glutamic acid,

(b) an amino acid at position corresponding to position 171 of the amino acid sequence represented by SEQ ID NO: 1 is substituted by glycine,

(c) an amino acid at position corresponding to position 269 of the amino acid sequence represented by SEQ ID NO: 1 is substituted by arginine, and

(d) an amino acid at position corresponding to position 298 of the amino acid sequence represented by SEQ ID NO: 1 is substituted by aspartic acid.

The IdeZ variant may have two to four amino acid variations, more preferably three or four amino acid variations, particularly preferably four amino acid variations, selected from the amino acid variations (a) to (d) in the amino acid sequence represented by SEQ ID NO: 1. In the case of selecting two or three of the amino acid variations (a) to (d), the combination thereof is arbitrary and preferably includes the amino acid variations (a) and (c), (a), (b) and (c), or (a), (c), and (d).

Particularly preferably, the IdeZ variant according to an embodiment comprises an amino acid sequence 90% or more, 95% or more, or 100% identical to the amino acid sequence represented by SEQ ID NO: 5, 9, or 13.

The IdeZ variant according to an embodiment may comprise an amino acid variation other than those mentioned above.

The IdeZ variant according to an embodiment, when produced using mammalian cells, preferably has 10 to 100 times, 10 to 70 times, or 10 to 40 times the protease activity of wild-type IdeZ. Since an IdeZ moiety contributes to the protease activity, an IdeZ variant-Fc region fusion protein mentioned later also has protease activity.

(2) Polynucleotide and expression vector

According to one embodiment, the present invention provides a polynucleotide encoding the IdeZ variant mentioned above. Those skilled in the art are capable of preparing the IdeZ variant according to an embodiment by use of a method known in the art on the basis of its nucleotide sequence. The IdeZ variant according to an embodiment may be synthesized by use of, for example, a gene synthesis method known in the art. Various methods known to those skilled in the art, such as an antibody gene synthesis method described in International Publication No. WO 90/07861, may be used as such gene synthesis methods.

According to a further embodiment, the present invention provides an expression vector comprising the polynucleotide mentioned above.

The expression vector according to an embodiment is not particularly limited as long as the polynucleotide can be produced in various host cells such as eukaryotic cells (e.g., animal cells, insect cells, plant cells, or yeasts) or prokaryotic cells (e.g., *E. coli*). Examples of such an expression vector include plasmid vectors and virus vectors. For example, pcDNA series (Thermo Fisher Scientific Inc.), pALTER(R)-MAX (Promega Corp.), pHEK293 Ultra Expression Vector (Takara Bio Inc.), or pEE6.4 or pEE12.4 (Lonza Biologics plc) can be used as the plasmid vector. For example, lentivirus, adenovirus, retrovirus, or adeno-associated virus can be used as the virus vector. For example, in the case of using lentivirus for introducing the polynucleotide according to an embodiment to cells, pLVSIN-CMV/EF1$\alpha$ vector (Takara Bio Inc.), pLenti vector (Thermo Fisher Scientific Inc.), or the like can be used as the lentivirus. In one embodiment, the vector for use in the expression vector according to an embodiment is pcDNA(TM)3.4-TOPO(R) (Thermo Fisher Scientific Inc.) and pcDNA(TM)3.1 (Thermo Fisher Scientific Inc.).

The expression vector according to an embodiment may contain a promoter operably linked to the polynucleotide mentioned above. Examples of the promoter for expressing the polynucleotide mentioned above in animal cells include promoters derived from viruses such as CMV, RSV, and SV40, actin promoter, EF (elongation factor) 1$\alpha$ promoter, and heat shock promoter. Examples of the promoter for expressing the polynucleotide mentioned above in a bacterium (e.g., a bacterium of the genus *Escherichia*) include trp promoter, lac promoter, $\lambda$PL promoter, and tac promoter. Examples of the promoter for expressing the polynucleotide mentioned above in a yeast include GAL1 promoter, GAL10 promoter, PH05 promoter, PGK promoter, GAP promoter, and ADH promoter.

In the case of using animal cells, insect cells, or a yeast as host cells, the expression vector according to an embodiment may contain a start codon and a stop codon. In this case, the expression vector may contain, for example, an enhancer sequence, 5' and 3' untranslated regions of a gene encoding the IdeZ variant according to an embodiment, a secretory signal sequence, a splicing junction, a polyadenylation site, or a replicable unit. In the case of using *E. coli* as host cells, the expression vector according to an embodiment may contain a start codon, a stop codon, a terminator region, and a replicable unit. The expression vector according to an embodiment may contain a drug selection marker gene (e.g., tetracycline resistance gene, ampicillin resistance gene, kanamycin resistance gene, neomycin resistance gene, or dihydrofolate reductase gene) usually used depending on a purpose.

(3) Host cell

According to one embodiment, the present invention provides a host cell transformed with the expression vector mentioned above. The host cell according to an embodiment may contain one or more polynucleotides according to an embodiment by transformation with the expression vector according to an embodiment.

The host cell to be transformed is not particularly limited as long as the host cell is compatible with the expression vector used and can express the polypeptide according to an embodiment by transformation with the expression vector. Various cells such as conventional cells usually used in the art or artificially established cells can be used as the host cell to be transformed. Examples thereof include animal cells (CHO-K1 cells, ExpiCHO-S(TM) cells, CHOK1SV cells, CHO-DG44 cells, HEK293 cells, NS0 cells, etc.), insect cells (Sf9, etc.), bacteria (bacteria of the genus *Escherichia,* etc.), and yeasts (the genus *Saccharomyces,* the genus *Pichia,* etc.). In one embodiment, the host cell is CHO-K1 cells or ExpiCHO-S cells.

A method for transforming the host cell is not particularly limited. For example, a method generally used by those skilled in the art, such as a calcium phosphate method, electroporation, or lipofection can be used.

The transformed host cell can be selected by a method generally used by those skilled in the art. For example, a cell isolation method such as a drug selection method using a drug selection marker gene and a drug such as tetracycline, ampicillin, neomycin, or hygromycin, a limiting dilution method, a single cell sorting method, or a colony pickup method can be used as the selection method.

(4) Method for producing IdeZ variant

Those skilled in the art are capable of preparing the IdeZ variant according to an embodiment by use of a method known in the art on the basis of sequence information and the like disclosed in the present specification. The IdeZ variant can be obtained, for example, by adding an amino acid variation to wild-type IdeZ by a method usually used in the art. Such a method may comprise, for example, the steps of: culturing the transformed host cell mentioned above so that the IdeZ variant is expressed into the host cell or a culture supernatant; and recovering, isolating, or purifying the IdeZ variant, though not limited to these methods as long as the IdeZ variant of interest is produced. Specific methods for culture, purification, and the like are as described in a method for producing a polypeptide mentioned later.

(5) Use

The IdeZ variant according to an embodiment can be employed in use heretofore known in the art about IgG cysteine protease. Such use is, for example, as described in the section Background Art. Examples thereof include use in high-quality recombinant protein production systems, use for suppressing rejection in transplantation, and use for the treatment of autoimmune diseases (Patent Literatures 1 and 2, WO2006/131347, WO2016/012285, etc.). Alternatively, possible use is, for example, the pretreatment of gene therapy using AAV (Patent Literature 1).

3. Polypeptide comprising IdeZ variant, Fc region variant, and polypeptide comprising Fc region variant

According to one embodiment, the present invention provides a polypeptide comprising the IdeZ variant mentioned above. The type of the polypeptide is not limited as long as the polypeptide comprises the IdeZ variant mentioned above. In one embodiment, the polypeptide comprising the IdeZ variant mentioned above is a fusion protein of the IdeZ variant mentioned above and an Fc region.

The origin of the Fc region is not particularly limited. For example, an Fc region of IgA1, IgA2, IgD, IgE, IgG1, IgG2, IgG3, IgG4, or IgM type exists as an antibody Fc region. The Fc region used may be derived from any of these antibodies and is preferably derived from IgG (IgG1, IgG2, IgG3, or IgG4), more preferably from human IgG, further preferably from human Igγ1, human Igγ2, human Igγ3, or human Igγ4, particularly preferably from human Igγ1.

The present inventors have found that use of an Fc region variant having a predetermined amino acid variation in the Fc region in the form of a fusion protein with the IdeZ variant mentioned above can suppress the self-digestion of the fusion protein. Accordingly, according to one embodiment, the present invention provides a fusion protein of the IdeZ variant and an Fc region variant. Since IdeZ is cysteine protease that acts on a hinge and cleaves this moiety, a possible problem with a fusion protein of the IdeZ linked to an Fc region is self-digestion by which the IdeZ acts on the hinge in its own fusion protein and cleaves it. Accordingly, the present inventors have focused on the properties of IdeZ or the like which has the action of cleaving a site between Fab and Fc of human IgG, but does not act on mouse IgG (IgG1 or IgG2b), and constructed a fusion protein by combining an Fc region rendered structurally similar to mouse IgG with the IdeZ variant. As a result, the self-digestion has been found to be suppressed.

Specifically, variations as described below are preferably added to the Fc region for use in the fusion protein of the IdeZ variant and the Fc region variant.

According to one embodiment, in the Fc region variant for use in the fusion protein, amino acid residue 236 is substituted by glutamic acid, and amino acid residue 237 is substituted by valine.

Preferably, in the Fc region variant, in addition, amino acid residue 234 is substituted by alanine, and amino acid residue 235 is substituted by glutamic acid.

More preferably, the Fc region variant comprises any one or more of the following amino acid variations: amino acid residue 227 is substituted by histidine, amino acid residue 228 is substituted by lysine, amino acid residue 233 is substituted by asparagine, and/or amino acid residue 238 is substituted by serine, in addition to those described above.

Further preferably, the Fc region variant comprises any one or more of the following amino acid variations: amino acid residue 253 is substituted by alanine, amino acid residue 293 or 294 is deleted, and/or amino acid residue 296 is substituted by alanine, in addition to those described above.

Particularly preferably, the Fc region variant is selected from the following Fc regions (1) and (2):

(1)

an Fc region variant in which
amino acid residue 234 is substituted by alanine,
amino acid residue 235 is substituted by glutamic acid,
amino acid residue 236 is substituted by glutamic acid,
amino acid residue 237 is substituted by valine,
amino acid residue 253 is substituted by alanine, and
amino acid residue 293 or 294 is deleted; and

(2)

an Fc region variant in which
amino acid residue 227 is substituted by histidine,
amino acid residue 228 is substituted by lysine,
amino acid residue 233 is substituted by asparagine,
amino acid residue 234 is substituted by alanine,

amino acid residue 235 is substituted by glutamic acid,
amino acid residue 236 is substituted by glutamic acid,
amino acid residue 237 is substituted by valine,
amino acid residue 238 is substituted by serine,
amino acid residue 253 is substituted by alanine, and
amino acid residue 293 or 294 is deleted.

More specifically, the Fc region variant comprises a sequence 90% or more, more preferably 95% or more, further preferably 100%, identical to a sequence selected from the group consisting of the amino acid sequences of Fc region moieties in the amino acid sequences of SEQ ID NOs: 16, 17, 21, and 22, i.e., a sequence consisting of amino acids at positions 316 to 541 in the amino acid sequence represented by SEQ ID NO: 16, a sequence consisting of amino acids at positions 316 to 541 in the amino acid sequence represented by SEQ ID NO: 17, a sequence consisting of amino acids at positions 316 to 541 in the amino acid sequence represented by SEQ ID NO: 21, and amino acids at positions 316 to 541 in the amino acid sequence represented by SEQ ID NO: 22. The Fc region variant having such a sequence is preferred in terms of a self-digestion suppressing effect. Particularly, the Fc region variant having the sequence of the Fc region moiety in SEQ ID NO: 16 or 21 or a sequence 90% or more, more preferably 95% or more, identical thereto is more preferred because few foreign substances are involved in production. Furthermore, the Fc region variant having the sequence of the Fc region moiety in SEQ ID NO: 16 or a sequence 90% or more, more preferably 95% or more, identical thereto is preferred because only a few number of amino acid variations are introduced and a high sialylation rate is obtained.

Particularly, amino acid variations L234A and L235A in a human Igγ1 constant region are called "LALA mutations". In this context, L234A is the substitution of leucine at position 234 according to the EU index in the human Igγ1 constant region by alanine. L235A is the substitution of leucine at position 235 according to the EU index in the human Igγ1 constant region by alanine. Amino acid variations L234A and L235E are called "LALE mutations". In this context, L235E is the substitution of leucine at position 235 according to the EU index in the human Igγ1 constant region by glutamic acid.

As mentioned later, for reducing ADA (anti-drug antibody) production, it is preferred to improve the sialylation rate of the Fc region. The deletion of an amino acid at position 293 or 294 contributes to improvement in sialylation rate. The Fc region variant preferably comprises the LALA mutations and the LALE mutations, in addition to those variations.

The Fc region variant may comprise a variation other than those mentioned above. Although such a variation is not particularly limited as long as desired effects can be achieved, the Fc region variant may comprise, for example, variations based on the knobs-into-holes technology (hereinafter, also referred to as "knobs-into-holes mutations"). The knobs-into-holes mutations are, for example, mutations that substitute an amino acid at position 366 of one Fc region in an Fc region dimer by tryptophane, and substitute, in the other Fc region, an amino acid at position 366 by serine, an amino acid at position 368 by alanine, and an amino acid at position 407 by valine (International Publication No. WO 1998/050431).

The IdeZ variant-Fe region (or Fc region variant) fusion product (i.e., fusion protein) may have a structure where one IdeZ variant is linked to each of the N termini of a complex of two Fc regions, or a one-armed structure where the IdeZ variant is linked to one of the Fc regions. In one embodiment, the fusion protein is a fusion protein of the IdeZ variant linked to the N terminus of the Fc region (or Fc region variant), and two such fusion proteins form a dimer. According to another embodiment, the fusion protein is a one-armed fusion protein comprising a fusion protein of the IdeZ variant linked to the N terminus of a first Fc region, and a second Fc region without the IdeZ variant linked thereto. As mentioned later, the Fc region in the fusion protein is preferably sialylated. The one-armed fusion protein comprising the Fc region variant is capable of achieving a relatively high sialylation rate without the use of glycosyltransferase for sialylation. Although a possible problem with the IdeZ-Fc region fusion protein is easy aggregation, the one-armed fusion protein is unlikely to cause such an aggregation problem.

In the case of the one-armed fusion protein, the first Fc region and the second Fc region can comprise the knobs-into-holes mutations mentioned above, CRIB, or BEAT (Vaccines 2021, 9 (7), 724). According to an embodiment of the present invention, the first Fc region and the second Fc region preferably comprise the knobs-into-holes mutations mentioned above.

According to one embodiment, the Fc region in the one-armed fusion protein comprises, in addition to the amino acid variations mentioned above, the following knobs-into-holes mutations (1) or (2):

(1)

in the first Fc region, amino acid residue 366 is substituted by tryptophane, and
in the second Fc region, amino acid residue 366 is substituted by serine, amino acid residue 368 is substituted by alanine, and amino acid residue 407 is substituted by valine; and

(2)

in the first Fc region, amino acid residue 366 is substituted by serine, amino acid residue 368 is substituted by alanine, and amino acid residue 407 is substituted by valine, and
in the second Fc region, amino acid residue 366 is substituted by tryptophane.

The fusion protein comprising the IdeZ variant mentioned above can be employed in use heretofore known in the art about IgG cysteine protease, as described in the above section "2. IdeZ variant (5) Use".

According to one embodiment, the present invention provides an Fc region variant itself suitable for the use mentioned above. Specific amino acid variations and the like are as mentioned above about the Fc region variant serving as a constituent of the fusion protein with the IdeZ variant.

According to one embodiment, the present invention also provides a polypeptide (fusion protein) comprising an additional protein and the Fc region variant mentioned above. Examples of the additional protein that can be used include, but are not limited to, antibodies, antigen binding fragments (single-chain variable fragment (scFv), Fab fragments, Fab' fragments, $F(ab')_2$ fragments, etc.) of antibodies, and bioactive peptides (e.g., receptors, adhesion molecules, ligands, and enzymes). The polypeptide comprising the Fc region variant is insusceptible to cleavage by IgG cysteine protease.

In one embodiment, the present invention also provides a fusion protein of IgG cysteine protease and the Fc region variant mentioned above. As described above, use of the Fc region variant can suppress the self-digestion of the fusion protein of the IdeZ variant and the Fc region variant. These effects can also be obtained in a fusion product of IgG cysteine protease other than the IdeZ variant. Such IgG cysteine protease is not limited as long as the IgG cysteine protease has the activity of cleaving IgG. Examples thereof include IdeS, IdeZ, IdeZ2, IdeE, IdeE2, IdeP, IdeSORK, and IgdE, and their variants. Among them, IdeZ or the aforementioned IdeZ variant according to an embodiment is preferred. In a certain embodiment, the fusion protein is a fusion protein of the IgG cysteine protease linked to the N terminus of the Fc region variant, and two such fusion proteins form a dimer. In a certain embodiment, the Fc region variant used comprises a sequence 90% or more, more preferably 95% or more, further preferably 100%, identical to a sequence selected from the group consisting of a sequence consisting of amino acids at positions 316 to 541 in the amino acid sequence represented by SEQ ID NO: 16, a sequence consisting of amino acids at positions 316 to 541 in the amino acid sequence represented by SEQ ID NO: 17, a sequence consisting of amino acids at positions 316 to 541 in the amino acid sequence represented by SEQ ID NO: 21, and amino acids at positions 316 to 541 in the amino acid sequence represented by SEQ ID NO: 22. According to another embodiment, the fusion protein is a one-armed fusion protein comprising a fusion protein of the IgG cysteine protease linked to the N terminus of a first Fc region, and a second Fc region without the IgG cysteine protease linked thereto. In a certain embodiment, in the case of a one-armed fusion protein, the first Fc region and the second Fc region comprise the knobs-into-holes mutations mentioned above.

4. Polynucleotide and expression vector

According to one embodiment, the present invention provides a polynucleotide encoding the polypeptide mentioned above. Those skilled in the art are capable of preparing the polynucleotide according to an embodiment by use of a method known in the art on the basis of its nucleotide sequence. The polynucleotide according to an embodiment may be synthesized by use of, for example, a gene synthesis method known in the art. Various methods known to those skilled in the art, such as an antibody gene synthesis method described in International Publication No. WO 90/07861, may be used as such gene synthesis methods.

According to a further embodiment, the present invention provides an expression vector comprising the polynucleotide mentioned above. In the case of the one-armed fusion protein, a polynucleotide encoding the fusion protein of the IdeZ variant linked to the N terminus of the first Fc region, and a polynucleotide encoding the one-armed fusion protein comprising the second Fc region without the IdeZ variant linked thereto may be separately contained in different vectors, or a plurality of polynucleotides may be contained in one vector.

The expression vector according to an embodiment is not particularly limited as long as the polynucleotide according to an embodiment can be produced in various host cells such as eukaryotic cells (e.g., animal cells, insect cells, plant cells, or yeasts) or prokaryotic cells (e.g., E. coli). Examples of such an expression vector include plasmid vectors and virus vectors. For example, pcDNA series (Thermo Fisher Scientific Inc.), pALTER(R)-MAX (Promega Corp.), pHEK293 Ultra Expression Vector (Takara Bio Inc.), or pEE6.4 or pEE12.4 (Lonza Biologics plc) can be used as the plasmid vector. For example, lentivirus, adenovirus, retrovirus, or adeno-associated virus can be used as the virus vector. For example, in the case of using lentivirus for introducing the polynucleotide according to an embodiment to cells, pLVSIN-CMV/EF1α vector (Takara Bio Inc.), pLenti vector (Thermo Fisher Scientific Inc.), or the like can be used as the lentivirus. In one embodiment, the vector for use in the expression vector according to an embodiment is pcDNA(TM)3.4-TOPO(R) (Thermo Fisher Scientific Inc.) and pcDNA(TM)3.1 (Thermo Fisher Scientific Inc.).

The expression vector according to an embodiment may contain a promoter operably linked to the polynucleotide of the present invention. Examples of the promoter for expressing the polynucleotide according to an embodiment in animal cells include promoters derived from viruses such as CMV, RSV, and SV40, actin promoter, EF (elongation factor) 1α promoter, and heat shock promoter. Examples of the promoter for expressing the polynucleotide according to an embodiment in a bacterium (e.g., a bacterium of the genus *Escherichia*) include trp promoter, lac promoter, λPL promoter, and tac promoter. Examples of the promoter for expressing the polynucleotide according to an embodiment in a yeast include GAL1 promoter, GAL10 promoter, PH05 promoter, PGK promoter, GAP promoter, and ADH promoter.

In the case of using animal cells, insect cells, or a yeast as host cells, the expression vector according to an embodiment may contain a start codon and a stop codon. In this case, the expression vector may contain, for example, an enhancer sequence, 5' and 3' untranslated regions of a gene encoding the Fc region variant according to an embodiment, a secretory signal sequence, a splicing junction, a polyadenylation site, or a replicable unit. In the case of using *E. coli* as host cells, the expression vector according to an embodiment may contain a start codon, a stop codon, a terminator region, and a replicable unit. The expression vector according to an embodiment may contain a drug selection marker gene (e.g., tetracycline resistance gene, ampicillin resistance gene, kanamycin resistance gene, neomycin resistance gene, or dihydrofolate reductase gene) usually used depending on a purpose.

5. Host cell

According to one embodiment, the present invention provides a host cell transformed with the expression vector mentioned above. The host cell according to an embodiment may contain one or more polynucleotides according to an embodiment by transformation with the expression vector according to an embodiment.

The host cell to be transformed is not particularly limited as long as the host cell is compatible with the expression vector used and can express the polypeptide according to an embodiment by transformation with the expression vector. Various cells such as conventional cells usually used in the art or artificially established cells can be used as the host cell to be transformed. Examples thereof include animal cells (CHO-K1 cells, ExpiCHO-S(TM) cells, CHOK1SV cells, CHO-DG44 cells, HEK293 cells, NS0 cells, etc.), insect cells (Sf9, etc.), bacteria (bacteria of the genus *Escherichia,* etc.), and yeasts (the genus *Saccharomyces,* the genus *Pichia,* etc.)). In one embodiment, the host cell is CHO-K1 cells or ExpiCHO-S cells.

A method for transforming the host cell is not particularly limited. For example, a method generally used by those skilled in the art, such as a calcium phosphate method, electroporation, or lipofection can be used.

The transformed host cell can be selected by a method generally used by those skilled in the art. For example, a cell isolation method such as a drug selection method using a drug selection marker gene and a drug such as tetracycline, ampicillin, neomycin, or hygromycin, a limiting dilution method, a single cell sorting method, or a colony pickup method can be used as the selection method.

6. Method for producing polypeptide

According to one embodiment, the present invention provides a method for producing the polypeptide comprising the IdeZ variant according to an embodiment. The method according to an embodiment may comprise, for example, the steps of: culturing the transformed host cell mentioned above so that the polypeptide is expressed into the host cell or a culture supernatant; and recovering, isolating, or purifying the polypeptide, though not limited to these methods as long as the polypeptide of interest is produced.

The transformed host cell can be cultured by a method known in the art. Culture conditions, for example, temperature, pH of a medium, and a culture time, are appropriately selected by those skilled in the art. When the host cell is animal cells, for example, MEM medium (Science, 1959, Vol. 130, p. 432-437), DMEM medium (Virol., 1959, Vol. 8, p. 396), RPMI-1640 medium (J. Am. Med. Assoc., 1967, Vol. 199, p. 519), or 199 medium (Exp. Biol. Med., 1950, Vol. 73, p. 1-8) containing approximately 5 to 20% fetal bovine serum can be used as the medium. The pH of the medium is, for example, approximately 6 to 8. The culture is usually performed at approximately 30 to 40°C for approximately 15 to 336 hours, if necessary, with aeration or stirring. When the host cell is insect cells, for example, Grace's medium (Proc. Natl. Acad. Sci. USA., 1985, Vol. 82, p. 8404) containing fetal bovine serum can be used as the medium. The pH of the medium is, for example, approximately 5 to 8. The culture is usually performed at approximately 20 to 40°C for approximately 15 to 100 hours, if necessary, with aeration or stirring.

When the host cell is *E. coli* or a yeast, for example, a liquid medium containing a nutrient is appropriate as the medium. The nutritive medium contains, for example, a carbon source and an inorganic nitrogen source or an organic nitrogen source necessary for the growth of the transformed host cell. Examples of the carbon source include glucose, dextran, soluble starch, and sucrose. Examples of the inorganic nitrogen source or the organic nitrogen source include ammonium salts, nitrates, amino acids, corn steep liquor, peptone, casein, meat extracts, soybean cake, and potato extracts. Other nutrients (e.g., inorganic salts (e.g., calcium chloride, sodium dihydrogen phosphate, and magnesium chloride) and vitamins) and antibiotics (e.g., tetracycline, neomycin, ampicillin, and kanamycin) may be contained, if desired. The pH of the medium is, for example, approximately 5 to 8. When the host cell is *E. coli,* for example, LB medium or M9 medium (Molecular Cloning, Cold Spring Harbor Laboratory, Vol. 3, A2.2) can be used as the medium. The culture is usually performed at approximately 14 to 43°C for approximately 3 to 24 hours, if necessary, with aeration or stirring. When the host cell is a yeast, for example, Burkholder minimum medium (Proc. Natl. Acad. Sci. USA., 1980, Vol. 77, p. 4505) can be used as the medium. The culture is usually performed at approximately 20 to 35°C for approximately 14 to 144 hours, if necessary, with aeration or stirring. The polypeptide of interest can be expressed by the culture as mentioned above.

The method according to an embodiment may further comprise the step of recovering, isolating, or purifying the polypeptide from the host cell, in addition to the step of culturing the transformed host cell so that the polypeptide comprising the IdeZ variant is expressed. Examples of the isolation or purification method include methods that exploit solubility, such as salting-out and solvent precipitation, methods that exploit difference in molecular weight, such as dialysis, ultrafiltration, and gel filtration, methods that exploit charge, such as ion exchange chromatography and hydroxyapatite chromatography, methods that exploit specific affinity, such as affinity chromatography, methods that exploit difference in hydrophobicity, such as reverse-phase high-performance liquid chromatography, and methods that exploit difference in isoelectric point, such as isoelectric focusing electrophoresis. In one embodiment, the polypeptide secreted into the culture supernatant can be purified by each chromatography, for example, column chromatography using a protein A column or a protein G column.

According to one embodiment, the present invention provides a polypeptide comprising the IdeZ variant, the polypeptide being produced by the production method mentioned above.

7. Sialylated fusion protein

The Fc region in the IdeZ variant-Fc region (or Fc region variant) fusion protein mentioned above may be sialylated (i.e., sialic acid may be added thereto). The sialylation of the Fc region can r ADA production against the fusion protein when the fusion protein is administered to the living body. The Fc region preferably has a variation so as to increase a sialylation rate. Examples of the Fc region comprising such a variation include Fc region variants comprising LALA mutations or LALE mutations and deletion of an amino acid at position 293 or 294. When amino acid residue 294 is deleted and the interaction between positions 234 and 235 is decreased by the LALA mutations or the LALE mutations, three-dimensional room such as space occurs in these sites. A further distortion in the sugar chain linked to asparagine at position 297 is thereby accepted, presumably resulting in a state in which sialic acid is easily added. Both the amino acids at positions 293 and 294 are usually glutamic acid in wild-type IgG. Therefore, the same variation can be obtained by deleting either of these amino acids. In one embodiment, in the Fc region variant, glutamic acid at position 293 or 294 is deleted. According to one embodiment, the present invention provides a sialylated IdeZ variant-Fc region (or Fc region variant) fusion protein.

The sialylation method is not particularly limited, and the sialylated fusion protein can be obtained by adding or expressing sialyltransferase in the step of culturing the host cell mentioned above. Examples of such an enzyme include β-galactoside α-2,6-sialyltransferase 1 (ST6GAL1), sialyltransferase, and β-galactoside α-2,3-sialyltransferase. This further increases the amount of sialic acid added to the Fc region. The sialyltransferase may be simply added, or an expression vector having an insert of DNA encoding this enzyme may be prepared such that the enzyme is co-expressed with a polypeptide such as an antibody. In the case of adding the sialyltransferase, the timing or the method of addition is not particularly limited. The sialyltransferase may be added, for example, in the course of polypeptide production by cell culture or may be added and reacted after polypeptide purification. In the case of expressing the sialyltransferase, examples thereof include, but are not limited to, a method of allowing cells to co-express a plasmid encoding the sialyltransferase and a plasmid encoding the polypeptide to produce a sialylated polypeptide. In the case of producing the polypeptide using cells containing endogenous glycosyltransferase, such as CHO cells, sialic acid can be added in the course of polypeptide production without the addition of the sialyltransferase as mentioned above.

The fusion protein comprising the Fc region variant comprising the variations so as to increase a sialylation rate as mentioned above has an increased amount of sialic acid added (i.e., degree of sialylation) as compared with sialylated wild-type Fc region or a sialylated Fc region variant having only the deletion of an amino acid at position 294. For example, the amount of sialic acid added to the sugar chain linked to asparagine at position 297 is increased. The amount of sialic acid added can be measured as described in Examples mentioned later, and indicated as a "sialylation rate (%)". As used herein, the "sialylation rate (%)" means the ratio of a sugar chain having a sialic acid added thereto to all sugar chains present in the Fc region, and a specific measurement method and calculation method are as described in Examples mentioned later.

The sialylated fusion protein according to an embodiment has a sialylation rate of, for example, 20 to 100%, 40 to 100%, 60 to 100%, 80 to 100%, or 90 to 100%.

The fusion protein and the sialylated fusion protein mentioned above can be employed in use heretofore known in the art about IgG cysteine protease. Such use is, for example, as described in the section Background Art. Examples thereof include use in high-quality recombinant protein production systems, use for suppressing rejection in transplantation, and use for the treatment of autoimmune diseases (Patent Literatures 1 and 2, WO2006/131347, WO2016/012285, etc.). Alternatively, the fusion protein thus prepared is advantageous in easy purification or modification and an adjustable half-life.

7. Pharmaceutical composition

According to one embodiment, the present invention provides a pharmaceutical composition comprising the IdeZ variant or the polypeptide mentioned above and a pharmaceutically acceptable carrier. The pharmaceutical composition can be prepared by a method usually used using an excipient usually used in the art, i.e., a pharmaceutical excipient, a pharmaceutical carrier, or the like. Examples of the dosage form of the pharmaceutical composition include parenteral formulations such as injections and formulations for intravenous drips. Administration such as intravenous administration, subcutaneous administration, or intraperitoneal administration can be performed. For the production of preparations, excipients, carriers, additives, and the like appropriate for these dosage forms can be used in a pharmaceutically acceptable range.

The pharmaceutical composition according to an embodiment may further comprise one or more additional therapeutic agents. The pharmaceutical composition according to an embodiment may be used in combination (concurrently or sequentially) with one or more additional therapeutic agents.

The amount of the IdeZ variant or the polypeptide according to an embodiment in the pharmaceutical composition can be appropriately set depending on the severity of symptoms or the age of a patient, the dosage form of the preparation used, etc.

The IdeZ variant or the polypeptide according to an embodiment and the pharmaceutical composition containing the same can be used in the suppression of rejection in organ transplantation, the treatment of autoimmune diseases, the pretreatment of gene therapy using AAV, and the like. According to another embodiment, the present invention provides a method for treating an autoimmune disease, comprising administering a therapeutically effective amount of the IdeZ variant or the polypeptide according to an embodiment to a subject. According to a further embodiment, the present invention includes the IdeZ variant or the polypeptide of the present invention for use in the treatment of an autoimmune disease. The present invention also provides use of the IdeZ variant or the polypeptide according to an embodiment in the production of a pharmaceutical composition for the treatment of an autoimmune disease. The autoimmune disease to be treated is not particularly limited.

Examples

<Example 1: Preparation of IdeZ-Fc fusion protein in mammalian cells and evaluation of activity>

A gene encoding an amino acid sequence from positions 35 to 349 of IdeZ protein derived from *Streptococcus equi* subsp. *zooepidemicus* (UniProt accession No: Q0PIW1, SEQ ID NO: 1) was linked at its 5' side to a nucleic acid sequence encoding an antibody heavy chain signal (Protein Eng., 1987, Vol. 1, p. 499-505) and at its 3' side to a nucleic acid sequence encoding a mouse Igγ1 constant region (from an amino acid glycine at position 223 to lysine at position 447 according to the EU index) harboring an amino acid mutation D265A and an amino acid mutation F328C. The obtained nucleic acid sequence was inserted into pcDNA3.4 vector (Thermo Fisher Scientific Inc.) to prepare an expression vector.

ExpiCHO-S cells (Thermo Fisher Scientific Inc.) were transfected with the obtained expression vector containing a nucleic acid sequence encoding an IdeZ-Fc fusion protein, and rotationally cultured for 4 days under conditions of 37°C and 8% $CO_2$. Then, a purified IdeZ-Fc fusion protein was obtained in accordance with a standard method. The obtained IdeZ-Fc fusion protein was designated as a wild-type IdeZ-mouse Fc fusion protein.

An IdeZ variant-Fc fusion protein was obtained by use of the same approach as above, by preparing an expression vector containing a nucleic acid sequence encoding a variant harboring four amino acid variations (T120E/N171G/S269R/N298D according to the numbering for SEQ ID NO: 1) in the IdeZ protein as a variant of the consensus sequence of an N-linked sugar chain. The obtained IdeZ variant-Fc fusion protein was designated as an IdeZ variant A-mouse Fc fusion protein.

The nucleotide sequence of the IdeZ in the wild-type IdeZ-mouse Fc fusion protein is shown in SEQ ID NO: 2, and the amino acid sequence encoded thereby is shown in SEQ ID NO: 3. The nucleotide sequence of the IdeZ variant in the IdeZ variant A-mouse Fc fusion protein is shown in SEQ ID NO: 4, and the amino acid sequence encoded thereby is shown in SEQ ID NO: 5.

Each fusion protein was heat-treated at 70°C for 10 minutes with NuPAGE LDS Sample Buffer (Life Technologies Corp., NP0008) and evaluated for its purity by SDS-PAGE using NuPAGE 4-12% Bis-Tris Gel (Life Technologies Corp., NP0322BOX). Reduction treatment was performed by the addition of NuPAGE Sample Reducing Agent (Life Technologies Corp., NP0009) during the heat treatment. The molecular weight marker used was Precision Plus Protein Unstained Standards (Bio-Rad Laboratories, Inc., 161-0363). The electrophoretic profile of each fusion protein is shown in Figure 1.

The results obtained under a non-reducing condition indicate the molecular weight of a homodimer and the presence of an aggregate, and the results obtained under a reducing condition indicate the molecular weight of a monomer.

In Figure 1, lane 5 depicts the molecular weight marker, lane 7 depicts the wild-type IdeZ-mouse Fc fusion protein, and lane 9 depicts the IdeZ variant A-mouse Fc fusion protein. A stronger and clearer band was detected at a position of a small molecular weight in the IdeZ variant A-mouse Fc fusion protein having the mutated consensus sequence of the N-linked sugar chain, as compared with the wild-type IdeZ-mouse Fc fusion protein. This suggests that the wild-type IdeZ-mouse Fc fusion protein had N-linked glycosylation. The IdeZ variant A-mouse Fc fusion protein was confirmed to be deglycosylated by the mutation in the consensus sequence of the N-linked sugar chain.

Next, each fusion protein was evaluated for its IgG protease activity by the cleavage evaluation of a human IgG1 antibody. The antibody to be cleaved was a human IgG1 antibody against KLH (keyhole limpet hemocyanin) which is absent *in vivo* (International Publication No. WO 2013/094723) (anti-KLH_hIgG1). To 10 μg of the antibody, each fusion protein was added in amounts of 1.0, 0.3, 0.1, 0.03, and 0.01 μg (final concentration ratios of the antibody to the fusion protein: 10, 33, 100, 333, and 1000 times, respectively). The final volume was set to 20 μL, and the antibody was treated at 37°C for 2 hours. The SDS-PAGE described above was carried out under a reducing condition. The electrophoretic profile is shown in Figure 2.

In Figure 2, arrow 1 depicts an H chain. Arrow 2 depicts a Fab' fragment. Lanes 3 to 7 depict the electrophoretic profiles of the antibody cleaved with the wild-type IdeZ-mouse Fc fusion protein at antibody concentration ratios of 10, 33, 100, 333, and 1000 times, respectively. Lanes 8 to 12 depict the electrophoretic profiles of the antibody tested in the same manner as above using the IdeZ variant A-mouse Fc fusion protein. Lane 13 depicts the electrophoretic profile of only anti-KLH_hIgG1.

Surprisingly, the wild-type IdeZ-mouse Fc fusion protein was confirmed to merely cleave the antibody up to the concentration ratio of the human IgG1 antibody to the fusion protein on the order of 33 times, whereas the IdeZ variant A-mouse Fc fusion protein was capable of cleaving the antibody up to the concentration ratio on the order of 1000 times. It was confirmed that the N-glycosylation of IdeZ to be expressed in mammalian cells brings about the attenuation of protease activity; and the mutation in the consensus sequence of the N-linked sugar chain improves the protease activity.

<Example 2: Preparation of 3-amino acid substitution form of IdeZ-Fc fusion protein and evaluation of activity>

Next, in order to confirm the influence of the consensus sequence of each N-linked sugar chain on the expression and activity of an IdeZ-Fc fusion protein in mammalian cells, IdeZ-Fc fusion proteins each having three amino acid variations: variant 1 (N171G/S269R/N298D) (hereinafter, referred to as an "IdeZ variant 1-mouse Fc fusion protein"), variant 2 (T120E/S269R/N298D) (hereinafter, referred to as an "IdeZ variant 2-mouse Fc fusion protein"), variant 3 (T120E/N171G/N298D) (hereinafter, referred to as an "IdeZ variant 3-mouse Fc fusion protein"), and variant 4 (T120E/N171G/S269R) (hereinafter, referred to as an "IdeZ variant 4-mouse Fc fusion protein") were prepared by the same method as in Example 1.

The nucleotide sequence of the IdeZ variant in the IdeZ variant 1-mouse Fc fusion protein is shown in SEQ ID NO: 6, and the amino acid sequence encoded thereby is shown in SEQ ID NO: 7. The nucleotide sequence of the IdeZ variant in the IdeZ variant 2-mouse Fc fusion protein is shown in SEQ ID NO: 8, and the amino acid sequence encoded thereby is shown in SEQ ID NO: 9. The nucleotide sequence of the IdeZ variant in the IdeZ variant 3-mouse Fc fusion protein is shown in SEQ ID NO: 10, and the amino acid sequence encoded thereby is shown in SEQ ID NO: 11. The nucleotide sequence of the IdeZ variant in the IdeZ variant 4-mouse Fc fusion protein is shown in SEQ ID NO: 12, and the amino acid sequence encoded thereby is shown in SEQ ID NO: 13.

The IdeZ variant 2-mouse Fc fusion protein, the IdeZ variant 3-mouse Fc fusion protein, and the IdeZ variant 4-mouse Fc fusion protein were able to produce purified proteins, whereas no purified protein was obtained from the IdeZ variant 1-mouse Fc fusion protein.

Subsequently, these proteins were evaluated for their purity by SDS-PAGE by the same method as in Example 1. The electrophoretic profile of each protein of the IdeZ variant 2-mouse Fc fusion protein, the IdeZ variant 3-mouse Fc fusion protein, and the IdeZ variant 4-mouse Fc fusion protein is shown in Figure 3. In Figure 3, lane 5 depicts the IdeZ variant 2-mouse Fc fusion protein, lane 7 depicts the IdeZ variant 3-mouse Fc fusion protein, and lane 9 depicts the IdeZ variant 4-mouse Fc fusion protein.

The IdeZ variant 3-mouse Fc fusion protein had a relatively large molecular weight, suggesting the glycosylation of asparagine at position 267.

Next, each protein was evaluated for its protease activity by the cleavage evaluation of a human IgG1 antibody by the same method as in Example 1. The electrophoretic profile is shown in Figure 4.

In Figure 4, arrow 1 depicts an H chain. Arrow 2 depicts a Fab' fragment. Lanes 3 to 7 of the left diagram depict the electrophoretic profiles of the antibody cleaved with the IdeZ variant 2-mouse Fc fusion protein at antibody concentration ratios of 10, 33, 100, 333, and 1000 times, respectively. Lanes 8 to 12 depict the electrophoretic profiles of the antibody tested in the same manner as above using the IdeZ variant 3-mouse Fc fusion protein. Lanes 3 to 7 of the right diagram depict the electrophoretic profiles of the antibody tested in the same manner as above using the IdeZ variant 4-mouse Fc fusion protein.

Interestingly, the IdeZ variant 2-mouse Fc fusion protein and the IdeZ variant 4-mouse Fc fusion protein were able to be confirmed to cleave the antibody at the concentration ratio of the human IgG1 antibody to the fusion protein on the order of 333 times to 1000 times, whereas the IdeZ variant 3-mouse Fc fusion protein was able to be confirmed to merely cleave the antibody up to the concentration ratio of the human IgG1 antibody to the fusion protein on the order of 33 times. From this result, mutations in the N-linked glycosylation consensus motifs at Asn118 and Asn267 of SEQ ID NO: 1 are effective for the expression of IdeZ in mammalian cells and the maintenance of the protease activity.

<Example 3-1: Preparation of bivalent IdeZ variant A-human Fc variant - 1>

In order to obtain a fusion product of IdeZ variant A and a human IgG1 Fc region, the self-digestion of which was suppressed, variant sequences of human Fc were designed. For the design, the sequences of mouse IgG 1 and IgG2b were referred to because these antibodies are not cleaved by IdeZ. Also, a Y296N mutation or a Y296R mutation reported as a mutation that hinders binding to IdeS was also referred to. Further, an E294del mutation (J. Immunol. 2019, Vol. 202, p. 1582-1594, and WO2016/016586) and an I253A mutation (Intl. Immunol., 2006, Vol. 18, p. 1759-1769) were introduced into each variant. Table 1 shows the name of each variant and its designed sequence.

[Table 1]

| Variant name | Variation introduced into human Fc | Amino acid | DNA |
|---|---|---|---|
| IdeZ variant A-human Fc variant 5 | E233N/L234A/L235E/I253A/E294del | SEQ ID NO: 14 | SEQ ID NO: 25 |
| IdeZ variant A-human Fc variant 6 | P227H/P228K/E233N/L234A/L235E/I253A/E294del | SEQ ID NO: 15 | SEQ ID NO: 26 |
| IdeZ variant A-human Fc variant 9 | L234A/L235E/I253A/E294del/Y296A | SEQ ID NO: 18 | SEQ ID NO: 29 |
| IdeZ variant A-human Fc variant 10 | L234A/L235E/I253A/E294del | SEQ ID NO: 19 | SEQ ID NO: 30 |
| IdeZ variant A-human Fc variant 11 | E233N/L234A/L235A/I253A/E294del/Y296A | SEQ ID NO: 20 | SEQ ID NO: 31 |

DNA encoding each IdeZ variant A-human Fc variant was synthesized in accordance with a routine method, linked at its 5' side to a nucleic acid sequence encoding an antibody heavy chain signal (Protein Eng., 1987, Vol. 1, p. 499-505), and inserted into pcDNA3.4 vector (Thermo Fisher Scientific Inc.) to prepare each IdeZ variant A-human Fc variant expression vector.

For the purpose of conferring $\alpha$2,6-linked sialic acid, ExpiCHO-S cells (Thermo Fisher Scientific Inc.) were transfected with an expression vector encoding $\beta$-1,4-galactosyltransferase 1 (B4GALT1) (UniProt accession No: P15291-1) and an expression vector encoding human $\beta$-galactoside $\alpha$-2,6-sialyltransferase 1 (ST6GAL1) (UniProt accession No: P15907-1), together with the expression vector encoding each IdeZ variant A-human Fc variant, and rotationally cultured for 4 days under conditions of 37°C and 8% $CO_2$. A culture supernatant was purified using MabSelect SuRe (Cytiva, 17-5438-02) in accordance with a routine method to obtain each IdeZ variant A-human Fc variant. The obtained IdeZ variant A-human Fc variant was designated as sialylated IdeZ variant A-human Fc variant. Each obtained sialylated IdeZ variant A-human Fc variant was analyzed by SDS-PAGE by the method described in Example 1. The electrophoretic profile of each sialylated IdeZ variant A-human Fc variant is shown in Figure 5.

In Figure 5, lane 1 depicts the molecular weight marker, lanes 2 to 6 depict the sialylated IdeZ variant A-human Fc variant 5, the sialylated IdeZ variant A-human Fc variant 6, the sialylated IdeZ variant A-human Fc variant 9, the sialylated IdeZ variant A-human Fc variant 10, and the sialylated IdeZ variant A-human Fc variant 11, respectively.

In the sialylated IdeZ variant A-human Fc variants 5, 6, 9, 10, and 11, a band presumably derived from the human Fc region was detected at a position between 25 kDa and 37 kDa of the molecular weight marker under a reducing condition, indicating that the sialylated IdeZ variant A-human Fc variants 5, 6, 9, 10, and 11 were self-digested.

<Example 3-2: Preparation of bivalent IdeZ variant A-human Fc variant - 2>

All the IdeZ variant A-human Fc variants designed in Example 3-1 were self-digested. Therefore, variant sequences of human Fc further harboring mutations were designed with reference to the sequences of mouse IgG1 and IgG2b. Table 2 shows the name of each variant and its designed sequence.

[Table 2]

| Variant name | Variation introduced into human Fc | Amino acid | DNA |
|---|---|---|---|
| IdeZ variant A-human Fc variant 7 | L234A/L235E/G236E/G237V/I253A/E294del | SEQ ID NO: 16 | SEQ ID NO: 27 |
| IdeZ variant A-human Fc variant 8 | P227H/P228K/E233N/L234A/L235E/G236E/G237V/P238S/I253A/E294del | SEQ ID NO: 17 | SEQ ID NO: 28 |
| IdeZ variant A-human Fc variant 17 | L234A/L235E/G236E/G237V/I253A/E294del/Y296A | SEQ ID NO: 21 | SEQ ID NO: 32 |
| IdeZ variant A-human Fc variant 18 | P227H/P228K/E233N/L234A/L235E/G236E/G237V/P238S/I253A/E294del/Y296A | SEQ ID NO: 22 | SEQ ID NO: 33 |

Each sialylated IdeZ variant A-human Fc variant described in Table 2 was obtained by the method described in Example 3-1.

In order to obtain each IdeZ variant A-human Fc variant prepared without co-expression with B4GALT1 and ST6GAL1, ExpiCHO-S cells (Thermo Fisher Scientific Inc.) were transfected with an expression vector encoding each IdeZ variant A-human Fc variant, and rotationally cultured for 4 days under conditions of 37°C and 8% $CO_2$. A culture supernatant was purified using MabSelect SuRe (Cytiva, 17-5438-02) in accordance with a routine method. The obtained IdeZ variant A-human Fc variant was designated as non-sialylated IdeZ variant A-human Fc variant.

The obtained sialylated IdeZ variant A-human Fc variants and the non-sialylated IdeZ variant A-human Fc variants (hereinafter, also simply referred to as "IdeZ variant A-human Fc variants") were analyzed by SDS-PAGE under reducing and non-reducing conditions. The electrophoretic profile of each IdeZ variant A-human Fc variant is shown in Figure 6.

In Figure 6, lane 1 depicts the molecular weight marker, and lanes 2 to 5 depict the non-sialylated IdeZ variant A-human Fc variants 7, 8, 17, and 18, respectively. Lanes 6 to 9 depict the sialylated IdeZ variant A-human Fc variants 7, 8, 17, and 18, respectively.

The results obtained under a reducing condition indicated that the IdeZ variant A-human Fc variant 7, 8, 17, or 18 was not self-digested. As seen from the results obtained under a non-reducing condition, in all the IdeZ variant A-human Fc variants, a band derived from the target was detected around 150 kDa of the molecular weight marker. In the IdeZ variant A-human Fc variant 8 and the IdeZ variant A-human Fc variant 18, a band other than the target was detected between 50 and 75 kDa of the molecular weight marker. By contrast, no such band was detected in the IdeZ variant A-human Fc variant 7 and the IdeZ variant A-human Fc variant 17. This indicated that the IdeZ variant A-human Fc variant 7 and the IdeZ variant A-human Fc variant 17 can produce the target with higher purity than that of the IdeZ variant A-human Fc variant 8 and the IdeZ variant A-human Fc variant 18.

<Example 4: Preparation of monovalent IdeZ variant A-human Fc variant>

As shown in Figure 6, a band was detected at a molecular weight higher than that of the target as a result of SDS-PAGE under a non-reducing condition. Therefore, the IdeZ variant A-human Fc variants were suspected of aggregation. In order to improve aggregating properties, variant sequences of human Fc further harboring mutations in the IdeZ variant A-human Fc variant 7 obtained in Example 3-2 were designed, and a monovalent IdeZ variant A-human Fc variant having the variant sequences of human Fc was designed. Table 3 shows the name of the variant, the names of the constituent chains, and the designed sequences.

[Table 3]

| Variant name | Variant constituent chain name | Variation introduced into human Fc | Amino acid | DNA |
|---|---|---|---|---|
| IdeZ variant A-human Fc variant 19 | IdeZ variant A-human Fc variant 19_holes | L234A/L235E/G236E/G237V/I253A/ E294del/T366S/L368A/Y407V | SEQ ID NO: 23 | SEQ ID NO: 34 |
| | human Fc variant 19_knobs | L234A/L235E/G236E/G237V/ I253A/E294del/T366W | SEQ ID NO: 24 | SEQ ID NO: 35 |

DNAs encoding the IdeZ variant A-human Fc variant 19_holes and the human Fc variant 19_knobs were synthesized in accordance with a routine method and each inserted into pcDNA3.4 vector (Thermo Fisher Scientific Inc.) to prepare each variant constituent chain expression vector. Cells were transfected with expression vectors encoding B4GALT1 and ST6GAL1, the IdeZ variant A-human Fc variant 19_holes expression vector, and the human Fc variant 19_knobs expression vector, together, by the method described in Example 3-1, and cultured. A culture supernatant was purified using MabSelect SuRe (Cytiva, 17-5438-02) in accordance with a routine method to obtain IdeZ variant A-human Fc variant. The obtained protein was designated as sialylated IdeZ variant A-human Fc variant 19. Also, ExpiCHO-S cells (Thermo Fisher Scientific Inc.) were transfected with the IdeZ variant A-human Fc variant 19_holes expression vector and the human Fc variant 19_knobs expression vector by the method described in Example 3-2, and cultured. A culture supernatant was purified using MabSelect SuRe (Cytiva, 17-5438-02) in accordance with a routine method to obtain non-sialylated IdeZ variant A-human Fc variant 19.

The obtained sialylated IdeZ variant A-human Fc variant 19 and non-sialylated IdeZ variant A-human Fc variant 19 were analyzed by SDS-PAGE under a reducing condition and under a non-reducing condition. Each electrophoretic profile is shown in Figure 7. In Figure 7, lane 1 depicts the molecular weight marker, lane 2 depicts the non-sialylated IdeZ variant A-human Fc variant 19, and lane 3 depicts the sialylated IdeZ variant A-human Fc variant 19.

Under a reducing condition, a band presumably derived from the human Fc variant 19_knobs was detected between 25 and 37 kDa of the molecular weight marker, and a band presumably derived from the IdeZ variant A-human Fc variant 19_holes was detected between 50 and 75 kDa of the molecular weight marker. Under a non-reducing condition, a band was detected around 100 kDa of the molecular weight marker. It was therefore confirmed that the target could be obtained.

<Example 5: Cleaving activity evaluation of IdeZ variant A-human Fc variant>

To 10 μg of anti-KLH_hIgG1 described in Example 1, the sialylated IdeZ variant A-human Fc variant 7 or the sialylated IdeZ variant A-human Fc variant 19 was added in amounts of 2, 0.4, 0.08, and 0.016 μg (final concentration ratios of the amounts of the sialylated IdeZ variant A-human Fc variant added to the amount of anti-KLH_hIgG1: 0.2, 0.04, 0.008, and 0.0016 times, respectively). The final volume was set to 20 μL, and the antibody was reacted at 37°C for 2 hours. The reaction product was analyzed by SDS-PAGE under a reducing condition. The electrophoretic profile is shown in Figure 8.

As a result, both the bivalent IdeZ variant A-human Fc variant 7 and the monovalent sialylated IdeZ variant A-human Fc variant 19 exhibited almost equivalent anti-KLH_hIgG1 cleaving activity.

<Example 6: Size-exclusion chromatography analysis>

In order to evaluate purity after affinity chromatography purification (purification using MabSelect SuRe (Cytiva, 17-5438-02)), the sialylated IdeZ variant A-human Fc variant 7 prepared in Example 3-2 and the sialylated IdeZ variant A-human Fc variant 19 prepared in Example 4 were each diluted into 1 mg/mL and analyzed using ACQUITY UPLC BEH200 SEC column (Waters Corp., #186005225). The results are shown in Table 4.

[Table 4]

| Sialylated IdeZ variant A-human Fc variant 7 | | | |
|---|---|---|---|
| Retention time (min) | Area (μV sec) | Area% | Height (μV) |
| 1.800 | 560848 | 6.79 | 82430 |
| 1.864 | 296574 | 3.59 | 73009 |

(continued)

| Sialylated IdeZ variant A-human Fc variant 7 | | | |
|---|---|---|---|
| Retention time (min) | Area ($\mu$V sec) | Area% | Height ($\mu$V) |
| 1.969 | 846832 | 10.25 | 143426 |
| 2.240 | 6380228 | 77.24 | 1301343 |
| 2.610 | 175933 | 2.13 | 10160 |

Sialylated IdeZ variant A-human Fc variant 19

| Retention time (min) | Area ($\mu$V sec) | Area% | Height ($\mu$V) |
|---|---|---|---|
| 1.794 | 226510 | 2.16 | 30656 |
| 1.937 | 248291 | 2.37 | 37410 |
| 2.055 | 857241 | 8.18 | 120414 |
| 2.265 | 277238 | 2.65 | 56646 |
| 2.393 | 7294906 | 69.63 | 1547522 |
| 2.633 | 1307459 | 12.48 | 180578 |
| 2.814 | 265092 | 2.53 | 40264 |

A peak of the target was detected at a retention time of 2.240 minutes for the sialylated IdeZ variant A-human Fc variant 7 and a retention time of 2.393 minutes for the sialylated IdeZ variant A-human Fc variant 19. In the sialylated IdeZ variant A-human Fc variant 7, peaks of 20.6 area% in total were detected at retention times shorter than that of the target. On the other hand, in the sialylated IdeZ variant A-human Fc variant 19, peaks of 15.4 area% in total were detected at retention times shorter than that of the target. This indicated that the sialylated IdeZ variant A-human Fc variant 19 had a low content of high-molecular-weightaggregates, as compared with the sialylated IdeZ variant A-human Fc variant 7.

<Example 7: Analysis of sialylation rates of various IdeZ variant A-human Fc variants>

The IdeZ variant A-human Fc variants obtained in Examples 3 and 4 were analyzed for their sialylation rates.

Example 7-1: Analysis of sialylation rates of sialylated IdeZ variant A-human Fc variants 5, 6, 9, 10, and 11

The sialylated IdeZ variant A-human Fc variants 5, 6, 9, 10, and 11 (hereinafter, also referred to as "uncleaved forms") obtained in Example 3-1 and their fragments formed by self-digestion (hereinafter, also referred to as "cleaved forms") were analyzed for their sialylation rates. To each sample, dithiothreitol (Thermo Fisher Scientific Inc., A39255; hereinafter, referred to as "DTT") was added, and incubated at 37°C for 1 hour to reduce an inter-chain disulfide bond. The sample thus reduced was injected into an HPLC system (Waters Corp.), separated through a reverse-phase column (Waters Corp., ACQUITY UPLC Protein BEH C4), and then introduced into a mass spectrometer (Waters Corp.). Sugar chains were attributed to their respective types from the difference between a theoretical molecular weight and the actually measured mass after reduction, and the proportion (%) of each sugar chain was calculated from an ionic strength ratio. The ratio of a sugar chain having a sialic acid added thereto to all sugar chains present in the Fc region was defined as the "sialylation rate (%)". The sialylation rate was calculated according to the following expression.

Sialylation rate (%) = (Total MS intensity of an H chain modified with the sialic acid-linked sugar chain) / (Total MS intensity of all the H chains) $\times$ 100.

The results are shown in Table 5.

The sialylated IdeZ variant A-human Fc variants 5, 6, 9, 10, and 11 and their cleaved forms were mostly sialylated. These results and the results of Example 3 suggested that self-digestion occurs during expression or purification in a manner dependent on a sequence rather than the presence or absence of sialylation for the sialylated IdeZ variant A-human Fc variants 5, 6, 9, 10, and 11.

[Table 5]

| Variant name | Sialylation rate of uncleaved form (%) | Sialylatio n rate of cleaved form (%) |
|---|---|---|
| Sialylated IdeZ variant A-human Fc variant 5 | 89.7 | 94.9 |
| Sialylated IdeZ variant A-human Fc variant 6 | 87.3 | 94.3 |
| Sialylated IdeZ variant A-human Fc variant 9 | 84.4 | 76.4 |
| Sialylated IdeZ variant A-human Fc variant 10 | 93.2 | 95.6 |
| Sialylated IdeZ variant A-human Fc variant 11 | Uncleaved form not detected | 77.1 |

Example 7-2: Analysis of sialylation rates of IdeZ variant A-human Fc variants 7, 8, 17, and 18
The IdeZ variant A-human Fc variants 7, 8, 17, and 18 obtained in Example 3-2 were analyzed for their sialylation rates by the method described in Example 7-1. The results are shown in Table 6.
As a result, the IdeZ variant A-human Fc variants 7, 8, 17, and 18 were found to be mostly sialylated by co-expression with the glycosyltransferase B4GALT1 and ST6GAL1.

[Table 6]

| Variant name | Sialylation rate (%) |
|---|---|
| Sialylated IdeZ variant A-human Fc variant 7 | 94.5 |
| Non-sialylated IdeZ variant A-human Fc variant 7 | 3.7 |
| Sialylated IdeZ variant A-human Fc variant 8 | 85.3 |
| Non-sialylated IdeZ variant A-human Fc variant 8 | 0.7 |
| Sialylated IdeZ variant A-human Fc variant 17 | 69.2 |
| Non-sialylated IdeZ variant A-human Fc variant 17 | 0.4 |
| Sialylated IdeZ variant A-human Fc variant 18 | 80.5 |
| Non-sialylated IdeZ variant A-human Fc variant 18 | 0.7 |

Example 7-3: Analysis of sialylation rate of IdeZ variant A-human Fc variant 19

The IdeZ variant A-human Fc variant 19 obtained in Example 4 was analyzed for its sialylation rate by the method described in Example 7-1. The IdeZ variant A-human Fc variant 19 was separated into the IdeZ variant A-human Fc variant 19_holes and the human Fc variant 19_knobs by reduction treatment, and each fragment was observed at a distinctive molecular weight in mass spectrometry. Therefore, their respective sialylation rates were calculated. The results are shown in Table 7.
As a result, both the fragments of the sialylated IdeZ variant A-human Fc variant 19 were found to be mostly sialylated by co-expression with the glycosyltransferase B4GALT1 and ST6GAL1. Surprisingly, the sialylation rates were relatively high without co-expression with the glycosyltransferase, as compared with the bivalent non-sialylated IdeZ variant A-human Fc variant 7.

[Table 7]

| Variant name | Sialylation rate of human Fc variant 19_knobs (%) | Sialylation rate of IdeZ variant A-human Fc variant 19_holes (%) |
|---|---|---|
| Sialylated IdeZ variant A-human Fc variant 19 | 97.1 | 97.4 |
| Non-sialylated IdeZ variant A-human Fc variant 19 | 60.3 | 27.9 |

In order to evaluate whether or not the sialic acid linking pattern would differ depending on the presence or absence of co-expression with the glycosyltransferase, analysis was conducted by the RapiFluor method. A sugar chain was cleaved out of the sample using GlycoWorks RapiFluor-MS N-Glycan Kit-96 Sample (Waters Corp., 176003606; hereinafter, referred to as "Kit") in accordance with the protocol of Kit and labeled with a fluorescent dye. The labeled sample was injected into an HPLC system (Waters Corp.), separated through an HILIC column (Waters Corp., ACQUITY UPLC Glycan BEH Amide), and then introduced into a mass spectrometer (Waters Corp.) and a fluorescence detector (Waters Corp.).

Sugar chains, including their linking patterns, were attributed by the comparison between a theoretical molecular weight and the actually measured mass and the comparison between the retention time of RapiFluor-MS Dextran Calibration Ladder (Waters Corp., 186007982) separately injected as a specimen and the actually measured retention time. The linking pattern ratio (%) of a typical sialylated sugar chain FA2G2S2 (Oxford notation) was calculated from a peak area value obtained with the fluorescence detector. The results are shown in Table 8.

These results indicated that a non-human $\alpha$-2,3 bond is preferential in the absence of co-expression with the glycosyltransferase while an $\alpha$-2,6 bond is preferential in the presence of co-expression with the glycosyltransferase. This suggested that the co-expression with the glycosyltransferase is effective for the preparation of a sialylated Fc region using CHO cells.

[Table 8]

| Variant name | $\alpha$-2,3 bond ratio of FA2G2S2 (%) | $\alpha$-2,6 bond ratio of FA2G2S2 (%) |
|---|---|---|
| Sialylated IdeZ variant A-human Fc variant 19 | 15 | 85 |
| Non-sialylated IdeZ variant A-human Fc variant 19 | 100 | Not detected |

The amino acid sequences and DNA sequences of the polypeptides according to an embodiment are shown in the following tables.

[Table 9-1]

Table 9

| SEQ ID NO: | Sequence name | Organism from which sequence is derived | DNA/protein | Sequence information |
|---|---|---|---|---|
| 1 | IdeZ protein | *Streptococcus equi* subsp. *Zooepidemicus* | Protein | MKTIAYPNKPHSLSAGLLTAIAIFSLASSNITYADDYQRNAAEVYAKEVPHQITSVWTK GVTPLTPEQFRYNNEDVIHAPYLAHQGWYDITKVFDGKDNLLCGAATAGNMLHWWF DQNKTEIEAYLSKHPEKQKIIFNNQELFDLKAAIDTKDSQTNSQLFNYFRDKAFPNLSAR QLGVMPDLVLDMFINGYYLNVFKTQSTDVNRPYQDKDKRGGIFDAVFTRGDQTTLLTA RHDLKNKGLNDISTIIKQELTEGRALALSHTYANVSISHVINLWGADFNAEGNLEAIYVT DSDANASIGMKKYFVGINAHGHVAISAKKIEGENIGAQVLGLFTLSSGKDIWQKLS |
| 2 | Wild-type IdeZ-mouse Fc fusion protein IdeZ | *Streptococcus equi* subsp. *Zooepidemicus* | DNA | GACGACTACCAGAGAGAAACGCCGCCGAGGTGTACGCCAAAGAGGTGCCACATCAGA TCACCTCCGTGTGGACCAAGGGCGTGACACCTCTGACACCCGAGCAGTTCCGGTAC AACAACGAGGATGTGATCCACGCTCCTTACCTGGCTCACCAAGGATGGTACGACAT CACCAAGGTGTTCGACGGCAAGGACAACCTGCTGTGTGGCGCTGCTACGGCCGGCA ATATGTGCACTGGTGGTTCGACCAGAAGATCATCTTCAACAATCAAGAGCTGTTCGAGC AAGCACCCCGAGAAGCAGCAGAAGATCATCTTCAACAATCAAGAGCTGTTCGACCTGAA GGCCGCGCCATCGACACCAAGGACTCCCAGACCAACAGCCAGCTGTTCAACTACTTCC GCGACAAGGCCTTTCCTAACCTGTCTGCTAGACCTGCTACTACCTGTGATGCCTGACCTGG TGCTGGACATGTTCATCAACGGCTACTACCTGAACGTGTTCAAGACCCAGTCCACCG ACGTGAACCGGCCTTACCAGGACAAGGATAAGAGAGGCGGCATCTTCGACGCCGTG TTCACCAGAGGCGATCAGACCACCATCCTCCACCATCAAGCAAGAGCTGACCGAGAACAA GGGCCTGAACGCCGACGACATCTCCACCATCCAGCCGCCAAGAAGATCGAGGGCGAGAGCCC TGGCTCTGTCTCACACCTACGCCAACGTGTCCATCAGCCACGTGATCAACCTGTGGG GCGCTGACTTTAACGCCGAGGGCAATCTGGAAGCCATCTACGTGACCGACTCTGAC GCCAACGCCTCCATCTCCGCCAAGAAGATCGAGGGCGAGAATATCGGCGGCCTCAACGCTCACGGCCA TGTGGCCATCTCCGCCAAGGATATCTGGCAGAAGGATATCTGGCAGAAGCTGTCTCGGTGCTGG GCCTGTTCACCCTGAGTTCCGGCAAGGATATCTGGCAGAAGCTGTCC |

[Table 9-2]

| Table 9 (continued) | | | | |
|---|---|---|---|---|
| 3 | Wild-type IdeZ-mouse Fc fusion protein IdeZ | *Streptococcus equi* subsp. *Zooepidemicus* | Protein | DDYQRNAAEVYAKEVPHQITSVWTKGVTPLTPEQFRYNNEDVIHAPYLAHQGWY DITKVFDGKDNLLCGAATAGNMLHWWFDQNKTEIEAYLSKHPEKQKIIFNNQELF DLKAAIDTKDSQTNSQLFNYFRDKAFPNLSARQLGVMPDLVLDMFINGYYLNVFK TQSTDVNRPYQDKDKRGGIFDAVFTRGDQTTLLTARHDLKNKGLNDISTIIKQELTE GRALALSHTYANVSISHVINLWGADFNAEGNLEAIYVTDSDANASIGMKKYFVGIN AHGHVAISAKKIEGENIGAQVLGLFTLSSGKDIWQKLS |
| 4 | IdeZ variant A-mouse Fc fusion protein IdeZ variant | *Streptococcus equi* subsp. *Zooepidemicus* | DNA | GACGACTACCAGAGAAACGCCGCCGAGGTGTACGCCAAAGAGGTGCCACATCA GATCACCTCCGTGTGGACCAAGGGCGTGACACCTCTGACACCCGAGCAGTTCCG GTACAACAACGAGGATGTGATCCACGCTCCTTACCTGGCTCACCAAGGATGGTA CGACATCACCAAGGTGTTCGACGGCAAGGACAACCTGCTGTGTGGCGCTGCTAC CGCCGGCAATATGCTGCACTGGTGGTTCGACCAGAACAAAGAGGAAATCGAGG CCTACCTGAGCAAGCACCCCGAGAAGCAGAAGATCATCTTCAACAATCAAGAG CTGTTCGACCTGAAGGCCGCCATCGACACCAAGGACTCCCAGACCAACAGCCA GCTGTTCAACTACTTCCGCGACAAGGCCTTTCCTGGCCTGTCCGCTAGACAGCT GGGAGTGATGCCTGACCTGGTGCTGGACATGTTCATCAACGGCTACTACCTGAA CGTGTTCAAGACCCAGTCCACCGACGTGAACCGGCCTTACCAGGACAAGGATA AGAGAGGCGGCATCTTCGACGCCGTGTTCACCAGAGGCGATCAGACCACACTG CTGACCGCCAGACACGATCTGAAGAACAAGGGCCTGAACGACATCTCCACCAT CATCAAGCAAGAGCTGACCGAGGGCAGAGCCCTGGCTCTGTCTCACACCTATGC CAACGTGCGGATCTCCCACGTGATCAACCTGTGGGGCGCTGACTTTAACGCCGA GGGCAATCTGGAAGCCATCTACGTGACCGACTCTGACGCCGATGCCTCCATCGG CATGAAGAAATACTTCGTGGGCATCAACGCCCACGGCCATGTGGCCATCTCTGC CAAGAAGATCGAGGGCGAGAATATCGGCGCTCAGGTGCTGGGCCTGTTCACCC TGAGTTCCGGCAAGGATATCTGGCAGAAGCTGTCC |

[Table 9-3]

Table 9 (continued)

| 5 | IdeZ variant A-mouse Fc fusion protein IdeZ variant | *Streptococcus equi* subsp. *Zooe-pidemicus* | Protein | DDYQRNAAEVYAKEVPHQITSVWTKGVTPLTPEQFRYNNEDVIHAPYLAHQGWY DITKVFDGKDNLLCGAATAGNMLHWWFDQNKEEIEAYLSKHPEKQKIIFNNQELF DLKAAIDTKDSQTNSQLFNYFRDKAFPGLSARQLGVMPDLVLDMFINGYYLNVFK TQSTDVNRPYQDKDKRGGIFDAVFTRGDQTTLLTARHDLKNKGLNDISTIIKQELTE GRALALSHTYANVRISHVINLWGADFNAEGNLEAIYVTDSDADASIGMKKYFVGIN AHGHVAISAKKIEGENIGAQVLGLFTLSSGKDIWQKLS |
|---|---|---|---|---|
| 6 | IdeZ variant 1-mouse Fc fusion protein IdeZ variant | *Streptococcus equi* subsp. *Zooe-pidemicus* | DNA | GACGACTACCAGAGAAACGCCGCCGAGGTGTACGCCAAAGAGGTGCCACATCA GATCACCTCCGTGTGGACCAAGGGCGTGACACCTCTGACACCCGAGCAGTTCCG GTACAACAACGAGGATGTGATCCACGCTCCTTACCTGGCTCACCAAGGATGGTA CGACATCACCAAGGTGTTCGACGGCAAGGACAACCTGCTGTGTGGCGCTGCTAC CGCCGGCAATATGCTGCACTGGTGGTTCGACCAGAACAAAacGGAAATCGAGGC CTACCTGAGCAAGCACCCCGAGAAGCAGAAGATCATCTTCAACAATCAAGAGC TGTTCGACCTGAAGGCCGCCATCGACACCAAGGACTCCCAGACCAACAGCCAG CTGTTCAACTACTTCCGCGACAAGGCCTTTCCTGGCCTGTCCGCTAGACAGCTG GGAGTGATGCCTGACCTGGTGCTGGACATGTTCATCAACGGCTACTACCTGAAC GTGTTCAAGACCCAGTCCACCGACGTGAACCGGCCTTACCAGGACAAGGATAA GAGAGGCGGCATCTTCGACGCCGTGTTCACCAGAGGCGATCAGACCACACTGC TGACCGCCAGACACGATCTGAAGAACAAGGGCCTGAACGACATCTCCACCATC ATCAAGCAAGAGCTGACCGAGGGCAGAGCCCTGGCTCTGTCTCACACCTATGCC AACGTGCGGATCTCCCACGTGATCAACCTGTGGGGCGCTGACTTTAACGCCGAG GGCAATCTGGAAGCCATCTACGTGACCGACTCTGACGCCGATGCCTCCATCGGC ATGAAGAAATACTTCGTGGGCATCAACGCCCACGGCCATGTGGCCATCTCTGCC AAGAAGATCGAGGGCGAGAATATCGGCGCTCAGGTGCTGGGCCTGTTCACCCT GAGTTCCGGCAAGGATATCTGGCAGAAGCTGTCC |

[Table 9-4]

| Table 9 (continued) | | | | |
|---|---|---|---|---|
| 7 | IdeZ variant 1-mouse Fc fusion protein IdeZ variant | *Streptococcus equi* subsp. *Zooe-pidemicus* | Protein | DDYQRNAAEVYAKEVPHQITSVWTKGVTPLTPEQFRYNNEDVIHAPYLAHQGWYDITKVFDGKDNLLCGAATAGNMLHWWFDQNKTEIEAYLSKHPEKQKIIFNNQELFDLKAAIDTKDSQTNSQLFNYFRDKAFPGLSARQLGVMPDLVLDMFINGYYLNVFKTQSTDVNRPYQDKDKRGGIFDAVFTRGDQTTLLTARHDLKNKGLNDISTIIKQELTEGRALALSHTYANVRISHVINLWGADFNAEGNLEAIYVTDSDADASIGMKKYFVGINAHGHVAISAKKIEGENIGAQVLGLFTLSSGKDIWQKLS |
| 8 | IdeZ variant 2-mouse Fc fusion protein IdeZ variant | *Streptococcus equi* subsp. *Zooe-pidemicus* | DNA | GACGACTACCAGAGAAACGCCGCCGAGGTGTACGCCAAAGAGGTGCCACATCAGATCACCTCCGTGTGGACCAAGGGCGTGACACCTCTGACACCCGAGCAGTTCCGGTACAACAACGAGGATGTGATCCACGCTCCTTACCTGGCTCACCAAGGATGGTACGACATCACCAAGGTGTTCGACGGCAAGGACAACCTGCTGTGTGGCGCTGCTACCGCCGGCAATATGCTGCACTGGTGGTTCGACCAGAACAAAGAGGAAATCGAGGCCTACCTGAGCAAGCACCCCGAGAAGCAGAAGATCATCTTCAACAATCAAGAGCTGTTCGACCTGAAGGCCGCCATCGACACCAAGGACTCCCAGACCAACAGCCAGCTGTTCAACTACTTCCGCGACAAGGCCTTTCCTaaCCTGTCCGCTAGACAGCTGGGAGTGATGCCTGACCTGGTGCTGGACATGTTCATCAACGGCTACTACCTGAACGTGTTCAAGACCCAGTCCACCGACGTGAACCGGCCTTACCAGGACAAGGATAAGAGAGGCGGCATCTTCGACGCCGTGTTCACCAGAGGCGATCAGACCACACTGCTGACCGCCAGACACGATCTGAAGAACAAGGGCCTGAACGACATCTCCACCATCATCAAGCAAGAGCTGACCGAGGGCAGAGCCCTGGCTCTGTCTCACACCTATGCCAACGTGCGGATCTCCCACGTGATCAACCTGTGGGGCGCTGACTTTAACGCCGAGGGCAATCTGGAAGCCATCTACGTGACCGACTCTGACGCCGATGCCTCCATCGGCATGAAGAAATACTTCGTGGGCATCAACGCCCACGGCCATGTGGCCATCTCTGCCAAGAAGATCGAGGGCGAGAATATCGGCGCTCAGGTGCTGGGCCTGTTCACCCTGAGTTCCGGCAAGGATATCTGGCAGAAGCTGTCC |

EP 4 772 624 A1

[Table 9-5]

| Table 9 (continued) | | | | |
|---|---|---|---|---|
| 9 | IdeZ variant 2-mouse Fc fusion protein IdeZ variant | *Streptococcus equi* subsp. *Zooepidemicus* | Protein | DDYQRNAAEVYAKEVPHQITSVWTKGVTPLTPEQFRYNNEDVIHAPYLAHQGWY DITKVFDGKDNLLCGAATAGNMLHWWFDQNKEEIEAYLSKHPEKQKIIFNNQELF DLKAAIDTKDSQTNSQLFNYFRDKAFPNLSARQLGVMPDLVLDMFINGYYLNVFK TQSTDVNRPYQDKDKRGGIFDAVFTRGDQTTLLTARHDLKNKGLNDISTIIKQELTE GRALALSHTYANVRISHVINLWGADFNAEGNLEAIYVTDSDADASIGMKKYFVGIN AHGHVAISAKKIEGENIGAQVLGLFTLSSGKDIWQKLS |
| 10 | IdeZ variant 3-mouse Fc fusion protein IdeZ variant | *Streptococcus equi* subsp. *Zooepidemicus* | DNA | GACGACTACCAGAGAAACGCCGCCGAGGTGTACGCCAAAGAGGTGCCACATCA GATCACCTCCGTGTGGACCAAGGGCGTGACACCTCTGACACCCGAGCAGTTCCG GTACAACAACGAGGATGTGATCCACGCTCCTTACCTGGCTCACCAAGGATGGTA CGACATCACCAAGGTGTTCGACGGCAAGGACAACCTGCTGTGTGGCGCTGCTAC CGCCGGCAATATGCTGCACTGGTGGTTCGACCAGAACAAAGAGGAAATCGAGG CCTACCTGAGCAAGCACCCCGAGAAGCAGAAGATCATCTTCAACAATCAAGAG CTGTTCGACCTGAAGGCCGCCATCGACACCAAGGACTCCCAGACCAACAGCCA GCTGTTCAACTACTTCCGCGACAAGGCCTTTCCTGGCCTGTCCGCTAGACAGCT GGGAGTGATGCCTGACCTGGTGCTGGACATGTTCATCAACGGCTACTACCTGAA CGTGTTCAAGACCCAGTCCACCGACGTGAACCGGCCTTACCAGGACAAGGATA AGAGAGGCGGCATCTTCGACGCCGTGTTCACCAGAGGCGATCAGACCACACTG CTGACCGCCAGACACGATCTGAAGAACAAGGGCCTGAACGACATCTCCACCAT CATCAAGCAAGAGCTGACCGAGGGCAGAGCCCTGGCTCTGTCTCACACCTATGC CAACGTGtcGATCTCCCACGTGATCAACCTGTGGGGCGCTGACTTTAACGCCGAG GGCAATCTGGAAGCCATCTACGTGACCGACTCTGACGCCGATGCCTCCATCGGC ATGAAGAAATACTTCGTGGGCATCAACGCCCACGGCCATGTGGCCATCTCTGCC AAGAAGATCGAGGGCGAGAATATCGGCGCTCAGGTGCTGGGCCTGTTCACCCT GAGTTCCGGCAAGGATATCTGGCAGAAGCTGTCC |

30

[Table 9-6]

| Table 9 (continued) | | | | |
|---|---|---|---|---|
| 11 | IdeZ variant 3-mouse Fc fusion protein IdeZ variant | Streptococcus equi subsp. Zooepidemicus | Protein | DDYQRNAAEVYAKEVPHQITSVWTKGVTPLTPEQFRYNNEDVIHAPYLAHQGWY DITKVFDGKDNLLCGAATAGNMLHWWFDQNKEEIEAYLSKHPEKQKIIFNNQELF DLKAAIDTKDSQTNSQLFNYFRDKAFPGLSARQLGVMPDLVLDMFINGYYLNVFK TQSTDVNRPYQDKDKRGGIFDAVFTRGDQTTLLTARHDLKNKGLNDISTIIKQELTE GRALALSHTYANVSISHVINLWGADFNAEGNLEAIYVTDSDADASIGMKKYFVGIN AHGHVAISAKKIEGENIGAQVLGLFTLSSGKDIWQKLS |
| 12 | IdeZ variant 4-mouse Fc fusion protein IdeZ variant | Streptococcus equi subsp. Zooepidemicus | DNA | GACGACTACCAGAGAAACGCCGCCGAGGTGTACGCCAAAGAGGTGCCACATCA GATCACCTCCGTGTGGACCAAGGGCGTGACACCTCTGACACCCGAGCAGTTCCG GTACAACAACGAGGATGTGATCCACGCTCCTTACCTGGCTCACCAAGGATGGTA CGACATCACCAAGGTGTTCGACGGCAAGGACAACCTGCTGTGTGGCGCTGCTAC CGCCGGCAATATGCTGCACTGGTGGTTCGACCAGAACAAAGAGGAAATCGAGG CCTACCTGAGCAAGCACCCCGAGAAGCAGAAGATCATCTTCAACAATCAAGAG CTGTTCGACCTGAAGGCCGCCATCGACACCAAGGACTCCCAGACCAACAGCCA GCTGTTCAACTACTTCCGCGACAAGGCCTTTCCTGGCCTGTCCGCTAGACAGCT GGGAGTGATGCCTGACCTGGTGCTGGACATGTTCATCAACGGCTACTACCTGAA CGTGTTCAAGACCCAGTCCACCGACGTGAACCGGCCTTACCAGGACAAGGATA AGAGAGGCGGCATCTTCGACGCCGTGTTCACCAGAGGCGATCAGACCACACTG CTGACCGCCAGACACGATCTGAAGAACAAGGGCCTGAACGACATCTCCACCAT CATCAAGCAAGAGCTGACCGAGGGCAGAGCCCTGGCTCTGTCTCACACCTATGC CAACGTGCGGATCTCCCACGTGATCAACCTGTGGGGCGCTGACTTTAACGCCGA GGGCAATCTGGAAGCCATCTACGTGACCGACTCTGACGCCaATGCCTCCATCGG CATGAAGAAATACTTCGTGGGCATCAACGCCCACGGCCATGTGGCCATCTCTGC CAAGAAGATCGAGGGCGAGAATATCGGCGCTCAGGTGCTGGGCCTGTTCACCC TGAGTTCCGGCAAGGATATCTGGCAGAAGCTGTCC |

EP 4 772 624 A1

[Table 9-7]

Table 9 (continued)

| 13 | IdeZ variant 4-mouse Fc fusion protein IdeZ variant | *Streptococcus equi* subsp. *Zooepidemicus* | Protein | DDYQRNAAEVYAKEVPHQITSVWTKGVTPLTPEQFRYNNEDVIHAPYLAHQGWY DITKVFDGKDNLLCGAATAGNMLHWWFDQNKEEIEAYLSKHPEKQKIIFNNQELF DLKAAIDTKDSQTNSQLFNYFRDKAFPGLSARQLGVMPDLVLDMFINGYYLNVFK TQSTDVNRPYQDKDKRGGIFDAVFTRGDQTTLLTARHDLKNKGLNDISTIIKQELTE GRALALSHTYANVRISHVINLWGADFNAEGNLEAIYVTDSDANASIGMKKYFVGIN AHGHVAISAKKIEGENIGAQVLGLFTLSSGKDIWQKLS |
| 14 | IdeZ variant A-human Fc variant 5 | Synthetic construct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCPPCPAPNAEGGPSVF LFPPKPKDTLMASRTPEVTCVVVDVSHEDPEVKFNWYVDGVEVHNAKTKPREQY NSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 15 | IdeZ variant A-human Fc variant 6 | Synthetic construct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCHKCPAPNAEGGPSVF LFPPKPKDTLMASRTPEVTCVVVDVSHEDPEVKFNWYVDGVEVHNAKTKPREQY NSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |

[Table 9-8]

Table 9 (continued)

| 16 | IdeZ variant A-human Fc variant 7 | Synthetic con-struct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCPPCPAPEAEEVPSVFL FPPKPKDTLMASRTPEVTCVVVDVSHEDPEVKFNWYVDGVEVHNAKTKPREQYNS TYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKL TVDKSRWQQGNVFSCSVMHEALHNHYTQKSLSLSPGK |
| --- | --- | --- | --- | --- |
| 17 | IdeZ variant A-human Fc variant 8 | Synthetic con-struct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCHKCPAPNAEEVSSVF LFPPKPKDTLMASRTPEVTCVVVDVSHEDPEVKFNWYVDGVEVHNAKTKPREQY NSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 18 | IdeZ variant A-human Fc variant 9 | Synthetic con-struct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCPPCPAPEAEGGPSVF LFPPKPKDTLMASRTPEVTCVVVDVSHEDPEVKFNWYVDGVEVHNAKTKPREQA NSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |

EP 4 772 624 A1

33

[Table 9-9]

Table 9 (continued)

| 19 | IdeZ variant A-human Fc variant 10 | Synthetic construct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCPPCPAPEAEGGPSVF LFPPKPKDTLMASRTPEVTCVVVDVSHEDPEVKFNWYVDGVEVHNAKTKPREQY NSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 20 | IdeZ variant A-human Fc variant 11 | Synthetic construct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCPPCPAPNAAGGPSVF LFPPKPKDTLMASRTPEVTCVVVDVSHEDPEVKFNWYVDGVEVHNAKTKPREQA NSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 21 | IdeZ variant A-human Fc variant 17 | Synthetic construct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCPPCPAPEAEEVPSVFL FPPKPKDTLMASRTPEVTCVVVDVSHEDPEVKFNWYVDGVEVHNAKTKPREQANS TYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKL TVDKSRWQQGNVFSCSVMHEALHNHYTQKSLSLSPGK |

[Table 9-10]

| Table 9 (continued) | | | | |
|---|---|---|---|---|
| 22 | IdeZ variant A-human Fc variant 18 | Synthetic construct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCHKCPAPNAEEVSSVF LFPPKPKDTLMASRTPEVTCVVVDVSHEDPEVKFNWYVDGVEVHNAKTKPREQA NSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 23 | IdeZ variant A-human Fc variant 19_holes | Synthetic construct | Protein | DDYQRNAAEVYAKEVPHQITSVWTKGVTPLTPEQFRYNNEDVIHAPYLAHQGWY DITKVFDGKDNLLCGAATAGNMLHWWFDQNKeEIEAYLSKHPEKQKIIFNNQELFD LKAAIDTKDSQTNSQLFNYFRDKAFPgLSARQLGVMPDLVLDMFINGYYLNVFKTQ STDVNRPYQDKDKRGGIFDAVFTRGDQTTLLTARHDLKNKGLNDISTIIKQELTEGR ALALSHTYANVrISHVINLWGADFNAEGNLEAIYVTDSDAdASIGMKKYFVGINAH GHVAISAKKIEGENIGAQVLGLFTLSSGKDIWQKLSDKTHTCPPCPAPEAEEVPSVFL FPPKPKDTLMASRTPEVTCVVVDVSHEDPEVKFNWYVDGVEVHNAKTKPREQYNS TYRVVSVLTVLHQDWLNGKEYKCKVSNKALPAPIEKTISKAKGQPREPQVYTLPPS RDELTKNQVSLSCAVKGFYPSDIAVEWESNGQPENNYKTTPPVLDSDGSFFLVSKL TVDKSRWQQGNVFSCSVMHEALHNHYTQKSLSLSPGK |
| 24 | human Fc variant 19_knobs | Synthetic construct | Protein | CPPCPAPEAEEVPSVFLFPPKPKDTLMASRTPEVTCVVVDVSHEDPEVKFNWYVDG VEVHNAKTKPREQYNSTYRVVSVLTVLHQDWLNGKEYKCKVSNKALPAPIEKTIS KAKGQPREPQVYTLPPSRDELTKNQVSLWCLVKGFYPSDIAVEWESNGQPENNYK TTPPVLDSDGSFFLYSKLTVDKSRWQQGNVFSCSVMHEALHNHYTQKSLSLSPGK |

[Table 9-11]

Table 9 (continued)

| 25 | IdeZ variant A-human Fc variant 5 | Synthetic construct | DNA | GACGACTACCAGAGAAATGCCGCCGAGGTGTACGCCAAAGAGGTGCCACATCAGA<br>TCACCTCCGTGTGGACCAAGGGCGTGACACCTCTGACACCCGAGCAGTTCCGGTAC<br>AACAACGAGGATGTGATCCACGCTCCTTACCTGGCTCACCAAGGATGGTACGACAT<br>CACCAAGGTGTTCGACGGCAAGGACAACCTGCTGTGTGGCGCTGCTACCGCCGGCA<br>ATATGCTGCACTGGTGGTTCGACCAGAACAAAGAGGAAATCGAGGCCTACCTGAGC<br>AAGCACCCCGAGAAGCAGAAGATCATCTTCAACAATCAAGAGCTGTTCGACCTGAA<br>GGCCGCCATCGACACCAAGGACTCCCAGACCAACAGCCAGCTGTTCAACTACTTCC<br>GCGACAAGGCCTTTCCTGGCCTGTCCGCTAGACAGCTGGGAGTGATGCCTGACCTG<br>GTGCTGGACATGTTCATCAACGGCTACTACCTGAACGTGTTCAAGACCCAGTCCACC<br>GACGTGAACCGGCCTTACCAGGACAAGGATAAGAGAGGCGGCATCTTCGACGCCGT<br>GTTCACCAGAGGCGATCAGACCACACTGCTGACCGCCAGACACGATCTGAAGAACA<br>AGGGCCTGAACGACATCTCCACCATCATCAAGCAAGAGCTGACCGAGGGCAGAGCC<br>CTGGCTCTGTCTCACACCTATGCCAACGTGCGGATCTCCCACGTGATCAACCTGTGG<br>GGCGCTGACTTTAACGCCGAGGGCAATCTGGAAGCCATCTACGTGACCGACTCTGA<br>CGCCGATGCCTCCATCGGCATGAAGAAATACTTCGTGGGCATCAACGCCCACGGCC<br>ATGTGGCCATCTCTGCCAAGAAGATCGAGGGCGAGAATATCGGCGCTCAGGTGCTG<br>GGCCTGTTCACCCTGAGTTCCGGCAAGGATATCTGGCAGAAGCTGTCCGACAAGAC<br>CCACACCTGTCCTCCATGTCCTGCTCCTAATGCTGAAGGCGGCCCTTCCGTGTTTCTG<br>TTCCCTCCAAAGCCTAAGGACACCCTGATGGCCTCTCGGACCCCTGAAGTGACATGC<br>GTGGTGGTGGATGTGTCCCACGAGGACCCAGAAGTGAAGTTCAATTGGTACGTGGA<br>CGGCGTGGAAGTGCACAACGCCAAGACCAAGCCTAGAGAGCAGTACAACTCCACCT<br>ACAGAGTGGTGTCCGTGCTGACCGTGCTGCACCAGGATTGGCTGAACGGCAAAGAG<br>TACAAGTGCAAGGTGTCCAACAAGGCCCTGCCTGCTCCAATCGAAAAGACCATCTC<br>CAAGGCCAAGGGCCAGCCTAGGGAACCCCAGGTTTACACCTTGCCTCCATCTCGGG<br>ACGAGCTGACCAAGAACCAGGTGTCCCTGACCTGTCTCGTGAAGGGCTTCTACCCCT<br>CCGATATCGCCGTGGAATGGGAGTCTAATGGCCAGCCAGAGAACAACTACAAGACA<br>ACCCCTCCTGTGCTGGACTCCGACGGCTCATTCTTTCTGTACTCCAAGCTGACAGTG<br>GACAAGTCCAGATGGCAGCAGGGCAACGTGTTCTCCTGCAGCGTGATGCACGAGGC<br>CCTGCACAATCACTACACACAGAAGTCCCTGTCTCTGTCCCCTGGCAAG |

[Table 9-12]

| Table 9 (continued) | | | | |
|---|---|---|---|---|
| 26 | IdeZ variant A-human Fc variant 6 | Synthetic construct | DNA | GACGACTACCAGAGAAATGCCGCCGAGGTGTACGCCAAAGAGGTGCCACATCAGATCACCTCCGTGTGGACCAAGGGCGTGACACCTCTGACACCCGAGCAGTTCCGGTACAACAACGAGGATGTGATCCACGCTCCTTACCTGGCTCACCAAGGATGGTACGACATCACCAAGGTGTTCGACGGCAAGGACAACCTGCTGTGTGGCGCTGCTACCGCCGGCAATATGCTGCACTGGTGGTTCGACCAGAACAAAGAGGAAATCGAGGCCTACCTGAGCAAGCACCCCGAGAAGCAGAAGATCATCTTCAACAATCAAGAGCTGTTCGACCTGAAGGCCGCCATCGACACCAAGGACTCCCAGACCAACAGCCAGCTGTTCAACTACTTCCGCGACAAGGCCTTTCCTGGCCTGTCCGCTAGACAGCTGGGAGTGATGCCTGACCTGGTGCTGGACATGTTCATCAACGGCTACTACCTGAACGTGTTCAAGACCCAGTCCACCGACGTGAACCGGCCTTACCAGGACAAGGATAAGAGAGGCGGCATCTTCGACGCCGTGTTCACCAGAGGCGATCAGACCACACTGCTGACCGCCAGACACGATCTGAAGAACAAGGGCCTGAACGACATCTCCACCATCATCAAGCAAGAGCTGACCGAGGGCAGAGCCCTGGCTCTGTCTCACACCTATGCCAACGTGCGGATCTCCCACGTGATCAACCTGTGGGGCGCTGACTTTAACGCCGAGGGCAATCTGGAAGCCATCTACGTGACCGACTCTGACGCCGATGCCTCCATCGGCATGAAGAAATACTTCGTGGGCATCAACGCCCACGGCCATGTGGCCATCTCTGCCAAGAAGATCGAGGGCGAGAATATCGGCGCTCAGGTGCTGGGCCTGTTCACCCTGAGTTCCGGCAAGGATATCTGGCAGAAGCTGTCCGACAAGACCCACACCTGTCACAAGTGCCCTGCTCCTAATGCTGAAGGCGGCCCTTCCGTGTTTCTGTTCCCTCCAAAGCCTAAGGACACCCTGATGGCCTCTCGGACCCCTGAAGTGACATGCGTGGTGGTGGATGTGTCCCACGAGGACCCAGAAGTGAAGTTCAATTGGTACGTGGACGGCGTGGAAGTGCACAACGCCAAGACCAAGCCTAGAGAGCAGTACAACTCCACCTACAGAGTGGTGTCCGTGCTGACCGTGCTGCACCAGGATTGGCTGAACGGCAAAGAGTACAAGTGCAAGGTGTCCAACAAGGCCCTGCCTGCTCCAATCGAAAAGACCATCTCCAAGGCCAAGGGCCAGCCTAGGGAACCCCAGGTTTACACCTTGCCTCCATCTCGGGACGAGCTGACCAAGAACCAGGTGTCCCTGACCTGTCTCGTGAAGGGCTTCTACCCCTCCGATATCGCCGTGGAATGGGAGTCTAATGGCCAGCCAGAGAACAACTACAAGACAACCCCTCCTGTGCTGGACTCCGACGGCTCATTCTTTCTGTACTCCAAGCTGACAGTGGACAAGTCCAGATGGCAGCAGGGCAACGTGTTCTCCTGCAGCGTGATGCACGAGGCCCTGCACAATCACTACACACAGAAGTCCCTGTCTCTGTCCCCTGGCAAG |

[Table 9-13]

| Table 9 (continued) | | | | |
|---|---|---|---|---|
| 27 | IdeZ variant A-human Fc variant 7 | Synthetic con-struct | DNA | GACGACTACCAGAGAAATGCCGCCGAGGTGTACGCCAAAGAGGTGCCACATCAGA TCACCTCCGTGTGGACCAAGGGCGTGACACCTCTGACACCCGAGCAGTTCCGGTAC AACAACGAGGATGTGATCCACGCTCCTTACCTGGCTCACCAAGGATGGTACGACAT CACCAAGGTGTTCGACGGCAAGGACAACCTGCTGTGTGGCGCTGCTACCGCCGGCA ATATGCTGCACTGGTGGTTCGACCAGAACAAAGAGGAAATCGAGGCCTACCTGAGC AAGCACCCCGAGAAGCAGAAGATCATCTTCAACAATCAAGAGCTGTTCGACCTGAA GGCCGCCATCGACACCAAGGACTCCCAGACCAACAGCCAGCTGTTCAACTACTTCC GCGACAAGGCCTTTCCTGGCCTGTCCGCTAGACAGCTGGGAGTGATGCCTGACCTG GTGCTGGACATGTTCATCAACGGCTACTACCTGAACGTGTTCAAGACCCAGTCCACC GACGTGAACCGGCCTTACCAGGACAAGGATAAGAGAGGCGGCATCTTCGACGCCGT GTTCACCAGAGGCGATCAGACCACACTGCTGACCGCCAGACACGATCTGAAGAACA AGGGCCTGAACGACATCTCCACCATCATCAAGCAAGAGCTGACCGAGGGCAGAGCC CTGGCTCTGTCTCACACCTATGCCAACGTGCGGATCTCCCACGTGATCAACCTGTGG GGCGCTGACTTTAACGCCGAGGGCAATCTGGAAGCCATCTACGTGACCGACTCTGA CGCCGATGCCTCCATCGGCATGAAGAAATACTTCGTGGGCATCAACGCCCACGGCC ATGTGGCCATCTCTGCCAAGAAGATCGAGGGCGAGAATATCGGCGCTCAGGTGCTG GGCCTGTTCACCCTGAGTTCCGGCAAGGATATCTGGCAGAAGCTGTCCGACAAGAC CCACACCTGTCCTCCATGTCCTGCTCCAGAGGCTGAAGAGGTGCCCTCCGTGTTTCT GTTCCCTCCAAAGCCTAAGGACACCCTGATGGCCTCTCGGACCCCTGAAGTGACAT GCGTGGTGGTGGATGTGTCCCACGAGGACCCAGAAGTGAAGTTCAATTGGTACGTG GACGGCGTGGAAGTGCACAACGCCAAGACCAAGCCTAGAGAGCAGTACAACTCCA CCTACAGAGTGGTGTCCGTGCTGACCGTGCTGCACCAGGATTGGCTGAACGGCAAA GAGTACAAGTGCAAGGTGTCCAACAAGGCCCTGCCTGCTCCTATCGAAAGACCAT CTCCAAGGCCAAGGGCCAGCCTAGGGAACCCCAGGTTTACACCTTGCCTCCATCTC GGGACGAGCTGACCAAGAACCAGGTGTCCCTGACCTGTCTCGTGAAGGGCTTCTAC CCCTCCGATATCGCCGTGGAATGGGAGTCTAATGGCCAGCCAGAGAACAACTACAA GACAACCCCTCCTGTGCTGGACTCCGACGGCTCATTCTTTCTGTACTCCAAGCTGAC AGTGGACAAGTCCAGATGGCAGCAGGGCAACGTGTTCTCCTGCAGCGTGATGCACG AGGCCCTGCACAATCACTACACACAGAAGTCCCTGTCTCTGTCCCCTGGCAAG |

EP 4 772 624 A1

[Table 9-14]

Table 9 (continued)

| 28 | IdeZ variant A-human Fc variant 8 | Synthetic construct | DNA | GACGACTACCAGAGAAATGCCGCCGAGGTGTACGCCAAAGAGGTGCCACATCAGA TCACCTCCGTGTGGACCAAGGGCGTGACACCTCTGACACCCGAGCAGTTCCGGTAC AACAACGAGGATGTGATCCACGCTCCTTACCTGGCTCACCAAGGATGGTACGACAT CACCAAGGTGTTCGACGGCAAGGACAACCTGCTGTGTGGCGCTGCTACCGCCGGCA ATATGCTGCACTGGTGGTTCGACCAGAACAAAGAGGAAATCGAGGCCTACCTGAGC AAGCACCCCGAGAAGCAGAAGATCATCTTCAACAATCAAGAGCTGTTCGACCTGAA GGCCGCCATCGACACCAAGGACTCCCAGACCAACAGCCAGCTGTTCAACTACTTCC GCGACAAGGCCTTTCCTGGCCTGTCCGCTAGACAGCTGGGAGTGATGCCTGACCTG GTGCTGGACATGTTCATCAACGGCTACTACCTGAACGTGTTCAAGACCCAGTCCACC GACGTGAACCGGCCTTACCAGGACAAGGATAAGAGAGGCGGCATCTTCGACGCCGT GTTCACCAGAGGCGATCAGACCACACTGCTGACCGCCAGACACGATCTGAAGAACA AGGGCCTGAACGACATCTCCACCATCATCAAGCAAGAGCTGACCGAGGGCAGAGCC CTGGCTCTGTCTCACACCTATGCCAACGTGCGGATCTCCCACGTGATCAACCTGTGG GGCGCTGACTTTAACGCCGAGGGCAATCTGGAAGCCATCTACGTGACCGACTCTGA CGCCGATGCCTCCATCGGCATGAAGAAATACTTCGTGGGCATCAACGCCCACGGCC ATGTGGCCATCTCTGCCAAGAAGATCGAGGGCGAGAATATCGGCGCTCAGGTGCTG GGCCTGTTCACCCTGAGTTCCGGCAAGGATATCTGGCAGAAGCTGTCCGACAAGAC CCACACCTGTCACAAGTGCCCTGCTCCTAACGCCGAAGAGGTGTCCTCCGTGTTTCT GTTCCCTCCAAAGCCTAAGGACACCCTGATGGCCTCTCGGACCCCTGAAGTGACAT GCGTGGTGGTGGATGTGTCCCACGAGGACCCAGAAGTGAAGTTCAATTGGTACGTG GACGGCGTGGAAGTGCACAACGCCAAGACCAAGCCTAGAGAGCAGTACAACTCCA CCTACAGAGTGGTGTCCGTGCTGACCGTGCTGCACCAGGATTGGCTGAACGGCAAA GAGTACAAGTGCAAGGTGTCCAACAAGGCCCTGCCTGCTCCAATCGAAAAGACCAT CTCCAAGGCCAAGGGCCAGCCTAGGGAACCCCAGGTTTACACCTTGCCTCCATCTC GGGACGAGCTGACCAAGAACCAGGTGTCCCTGACCTGTCTCGTGAAGGGCTTCTAC CCCTCCGATATCGCCGTGGAATGGGAGTCTAATGGCCAGCCAGAGAACAACTACAA GACAACCCCTCCTGTGCTGGACTCCGACGGCTCATTCTTTCTGTACTCCAAGCTGAC AGTGGACAAGTCCAGATGGCAGCAGGGCAACGTGTTCTCCTGCAGCGTGATGCACG AGGCCCTGCACAATCACTACACACAGAAGTCCCTGTCTCTGTCCCCTGGCAAG |

[Table 9-15]

Table 9 (continued)

| 29 | IdeZ variant A-human Fc variant 9 | Synthetic construct | DNA | GACGACTACCAGAGAAATGCCGCCGAGGTGTACGCCAAAGAGGTGCCACATCAGATCACCTCCGTGTGGACCAAGGGCGTGACACCTCTGACACCCGAGCAGTTCCGGTACAACAACGAGGATGTGATCCACGCTCCTTACCTGGCTCACCAAGGATGGTACGACATCACCAAGGTGTTCGACGGCAAGGACAACCTGCTGTGTGGCGCTGCTACCGCCGGCAATATGCTGCACTGGTGGTTCGACCAGAACAAAGAGGAAATCGAGGCCTACCTGAGCAAGCACCCCGAGAAGCAGAAGATCATCTTCAACAATCAAGAGCTGTTCGACCTGAAGGCCGCCATCGACACCAAGGACTCCCAGACCAACAGCCAGCTGTTCAACTACTTCCGCGACAAGGCCTTTCCTGGCCTGTCCGCTAGACAGCTGGGAGTGATGCCTGACCTGGTGCTGGACATGTTCATCAACGGCTACTACCTGAACGTGTTCAAGACCCAGTCCACCGACGTGAACCGGCCTTACCAGGACAAGGATAAGAGAGGCGGCATCTTCGACGCCGTGTTCACCAGAGGCGATCAGACCACACTGCTGACCGCCAGACACGATCTGAAGAACAAGGGCCTGAACGACATCTCCACCATCATCAAGCAAGAGCTGACCGAGGGCAGAGCCCTGGCTCTGTCTCACACCTATGCCAACGTGCGGATCTCCCACGTGATCAACCTGTGGGGCGCTGACTTTAACGCCGAGGGCAATCTGGAAGCCATCTACGTGACCGACTCTGACGCCGATGCCTCCATCGGCATGAAGAAATACTTCGTGGGCATCAACGCCCACGGCCATGTGGCCATCTCTGCCAAGAAGATCGAGGGCGAGAATATCGGCGCTCAGGTGCTGGGCCTGTTCACCCTGAGTTCCGGCAAGGATATCTGGCAGAAGCTGTCCGACAAGACCCACACCTGTCCTCCATGTCCTGCTCCAGAAGCTGAAGGCGGCCCTTCCGTGTTTCTGTTCCCTCCAAAGCCTAAGGACACCCTGATGGCCTCTCGGACCCCTGAAGTGACATGCGTGGTGGTGGATGTGTCCCACGAGGATCCCGAAGTGAAGTTCAATTGGTACGTGGACGGCGTGGAAGTGCACAACGCCAAGACCAAGCCTAGAGAGCAGGCCAACTCCACCTACAGAGTGGTGTCTGTGCTGACCGTGCTGCACCAGGATTGGCTGAACGGCAAAGAGTACAAGTGCAAGGTGTCCAACAAGGCCCTGCCTGCTCCTATCGAAAAGACCATCTCCAAGGCCAAGGGCCAGCCTAGGGAACCCCAGGTTTACACCTTGCCACCTTCTCGGGACGAGCTGACCAAGAACCAGGTGTCCCTGACCTGTCTGGTCAAGGGCTTCTACCCCTCCGATATCGCCGTGGAATGGGAGTCTAATGGCCAGCCTGAGAACAACTACAAGACAACCCCTCCTGTGCTGGACTCCGACGGCTCATTCTTTCTGTACTCCAAGCTGACAGTGGACAAGTCCAGATGGCAGCAGGGCAACGTGTTCTCCTGCAGCGTGATGCACGAGGCCCTGCACAATCACTACACCCAGAAGTCCCTGTCTCTGTCCCCTGGCAAG |

[Table 9-16]

| | | | | |
|---|---|---|---|---|
| | Table 9 (continued) | | | |
| 30 | IdeZ variant A-human Fc variant 10 | Synthetic construct | DNA | GACGACTACCAGAGAAATGCCGCCGAGGTGTACGCCAAAGAGGTGCCACATCAGA TCACCTCCGTGTGGACCAAGGGCGTGACACCTCTGACACCCGAGCAGTTCCGGTAC AACAACGAGGATGTGATCCACGCTCCTTACCTGGCTCACCAAGGATGGTACGACAT CACCAAGGTGTTCGACGGCAAGGACAACCTGCTGTGTGGCGCTGCTACCGCCGGCA ATATGCTGCACTGGTGGTTCGACCAGAACAAAGAGGAAATCGAGGCCTACCTGAGC AAGCACCCCGAGAAGCAGAAGATCATCTTCAACAATCAAGAGCTGTTCGACCTGAA GGCCGCCATCGACACCAAGGACTCCCAGACCAACAGCCAGCTGTTCAACTACTTCC GCGACAAGGCCTTTCCTGGCCTGTCCGCTAGACAGCTGGGAGTGATGCCTGACCTG GTGCTGGACATGTTCATCAACGGCTACTACCTGAACGTGTTCAAGACCCAGTCCACC GACGTGAACCGGCCTTACCAGGACAAGGATAAGAGAGGCGGCATCTTCGACGCCGT GTTCACCAGAGGCGATCAGACCACACTGCTGACCGCCAGACACGATCTGAAGAACA AGGGCCTGAACGACATCTCCACCATCATCAAGCAAGAGCTGACCGAGGGCAGAGCC CTGGCTCTGTCTCACACCTATGCCAACGTGCGGATCTCCCACGTGATCAACCTGTGG GGCGCTGACTTTAACGCCGAGGGCAATCTGGAAGCCATCTACGTGACCGACTCTGA CGCCGATGCCTCCATCGGCATGAAGAAATACTTCGTGGGCATCAACGCCCACGGCC ATGTGGCCATCTCTGCCAAGAAGATCGAGGGCGAGAATATCGGCGCTCAGGTGCTG GGCCTGTTCACCCTGAGTTCCGGCAAGGATATCTGGCAGAAGCTGTCCGACAAGAC CCACACCTGTCCTCCATGTCCTGCTCCAGAAGCTGAAGGCGGCCCTTCCGTGTTTCT GTTCCCTCCAAAGCCTAAGGACACCCTGATGGCCTCTCGGACCCCTGAAGTGACAT GCGTGGTGGTGGATGTGTCCCACGAGGATCCCGAAGTGAAGTTCAATTGGTACGTG GACGGCGTGGAAGTGCACAACGCCAAGACCAAGCCTAGAGAGCAGTACAACTCCA CCTACAGAGTGGTGTCCGTGCTGACCGTGCTGCACCAGGATTGGCTGAACGGCAAA GAGTACAAGTGCAAGGTGTCCAACAAGGCCCTGCCTGCTCCTATCGAAAGACCAT CTCCAAGGCCAAGGGCCAGCCTAGGGAACCCCAGGTTTACACCTTGCCACCTTCTC GGGACGAGCTGACCAAGAACCAGGTGTCCCTGACCTGTCTGGTCAAGGGCTTCTAC CCCTCCGATATCGCCGTGGAATGGGAGTCTAATGGCCAGCCTGAGAACAACTACAA GACAACCCCTCCTGTGCTGGACTCCGACGGCTCATTCTTTCTGTACTCCAAGCTGAC AGTGGACAAGTCCAGATGGCAGCAGGGCAACGTGTTCTCCTGCAGCGTGATGCACG AGGCCCTGCACAATCACTACACCCAGAAGTCCCTGTCTCTGTCCCCTGGCAAG |

EP 4 772 624 A1

[Table 9-17]

| Table 9 (continued) | | | | |
|---|---|---|---|---|
| 31 | IdeZ variant A-human Fc variant 11 | Synthetic construct | DNA | GACGACTACCAGAGAAATGCCGCCGAGGTGTACGCCAAAGAGGTGCCACATCAGATCACCTCCGTGTGGACCAAGGGCGTGACACCTCTGACACCCGAGCAGTTCCGGTACAACAACGAGGATGTGATCCACGCTCCTTACCTGGCTCACCAAGGATGGTACGACATCACCAAGGTGTTCGACGGCAAGGACAACCTGCTGTGTGGCGCTGCTACCGCCGGCAATATGCTGCACTGGTGGTTCGACCAGAACAAAGAGGAAATCGAGGCCTACCTGAGCAAGCACCCCGAGAAGCAGAAGATCATCTTCAACAATCAAGAGCTGTTCGACCTGAAGGCCGCCATCGACACCAAGGACTCCCAGACCAACAGCCAGCTGTTCAACTACTTCCGCGACAAGGCCTTTCCTGGCCTGTCCGCTAGACAGCTGGGAGTGATGCCTGACCTGGTGCTGGACATGTTCATCAACGGCTACTACCTGAACGTGTTCAAGACCCAGTCCACCGACGTGAACCGGCCTTACCAGGACAAGGATAAGAGAGGCGGCATCTTCGACGCCGTGTTCACCAGAGGCGATCAGACCACACTGCTGACCGCCAGACACGATCTGAAGAACAAGGGCCTGAACGACATCTCCACCATCATCAAGCAAGAGCTGACCGAGGGCAGAGCCCTGGCTCTGTCTCACACCTATGCCAACGTGCGGATCTCCCACGTGATCAACCTGTGGGGCGCTGACTTTAACGCCGAGGGCAATCTGGAAGCCATCTACGTGACCGACTCTGACGCCGATGCCTCCATCGGCATGAAGAAATACTTCGTGGGCATCAACGCCCACGGCCATGTGGCCATCTCTGCCAAGAAGATCGAGGGCGAGAATATCGGCGCTCAGGTGCTGGGCCTGTTCACCCTGAGTTCCGGCAAGGATATCTGGCAGAAGCTGTCCGACAAGACCCACACCTGTCCTCCATGTCCTGCTCCTAATGCTGCTGGCGGCCCTTCCGTGTTTCTGTTCCCTCCAAAGCCTAAGGACACCCTGATGGCCTCTCGGACCCCTGAAGTGACATGCGTGGTGGTGGATGTGTCCCACGAGGATCCCGAAGTGAAGTTCAATTGGTACGTGGACGGCGTGGAAGTGCACAACGCCAAGACCAAGCCTAGAGAGCAGGCCAACTCCACCTACAGAGTGGTGTCTGTGCTGACCGTGCTGCACCAGGATTGGCTGAACGGCAAAGAGTACAAGTGCAAGGTGTCCAACAAGGCCCTGCCTGCTCCAATCGAAAAGACCATCTCCAAGGCCAAGGGCCAGCCTAGGGAACCCCAGGTTTACACCTTGCCACCTTCTCGGGACGAGCTGACCAAGAACCAGGTGTCCCTGACCTGTCTGGTCAAGGGCTTCTACCCCTCCGATATCGCCGTGGAATGGGAGTCTAATGGCCAGCCTGAGAACAACTACAAGACAACCCCTCCTGTGCTGGACTCCGACGGCTCATTCTTTCTGTACTCCAAGCTGACAGTGGACAAGTCCAGATGGCAGCAGGGCAACGTGTTCTCCTGCAGCGTGATGCACGAGGCCCTGCACAATCACTACACCCAGAAGTCCCTGTCTCTGTCCCCTGGCAAG |

EP 4 772 624 A1

[Table 9-18]

| Table 9 (continued) | | | | |
|---|---|---|---|---|
| 32 | IdeZ variant A-human Fc variant 17 | Synthetic construct | DNA | GACGACTACCAGAGAAATGCCGCCGAGGTGTACGCCAAAGAGGTGCCACATCAGA TCACCTCCGTGTGGACCAAGGGCGTGACACCTCTGACACCCGAGCAGTTCCGGTAC AACAACGAGGATGTGATCCACGCTCCTTACCTGGCTCACCAAGGATGGTACGACAT CACCAAGGTGTTCGACGGCAAGGACAACCTGCTGTGTGGCGCTGCTACCGCCGGCA ATATGCTGCACTGGTGGTTCGACCAGAACAAAGAGGAAATCGAGGCCTACCTGAGC AAGCACCCCGAGAAGCAGAAGATCATCTTCAACAATCAAGAGCTGTTCGACCTGAA GGCCGCCATCGACACCAAGGACTCCCAGACCAACAGCCAGCTGTTCAACTACTTCC GCGACAAGGCCTTTCCTGGCCTGTCCGCTAGACAGCTGGGAGTGATGCCTGACCTG GTGCTGGACATGTTCATCAACGGCTACTACCTGAACGTGTTCAAGACCCAGTCCACC GACGTGAACCGGCCTTACCAGGACAAGGATAAGAGAGGCGGCATCTTCGACGCCGT GTTCACCAGAGGCGATCAGACCACACTGCTGACCGCCAGACACGATCTGAAGAACA AGGGCCTGAACGACATCTCCACCATCATCAAGCAAGAGCTGACCGAGGGCAGAGCC CTGGCTCTGTCTCACACCTATGCCAACGTGCGGATCTCCCACGTGATCAACCTGTGG GGCGCTGACTTTAACGCCGAGGGCAATCTGGAAGCCATCTACGTGACCGACTCTGA CGCCGATGCCTCCATCGGCATGAAGAAATACTTCGTGGGCATCAACGCCCACGGCC ATGTGGCCATCTCTGCCAAGAAGATCGAGGGCGAGAATATCGGCGCTCAGGTGCTG GGCCTGTTCACCCTGAGTTCCGGCAAGGATATCTGGCAGAAGCTGTCCGACAAGAC CCACACCTGTCCTCCATGTCCTGCTCCAGAGGCTGAAGAGGTGCCCTCCGTGTTTCT GTTCCCTCCAAAGCCTAAGGACACCCTGATGGCCTCTCGGACCCCTGAAGTGACAT GCGTGGTGGTGGATGTGTCCCACGAGGACCCAGAAGTGAAGTTCAATTGGTACGTG GACGGCGTGGAAGTGCACAACGCCAAGACCAAGCCTAGAGAGCAGgccAACTCCAC CTACAGAGTGGTGTCCGTGCTGACCGTGCTGCACCAGGATTGGCTGAACGGCAAAG AGTACAAGTGCAAGGTGTCCAACAAGGCCCTGCCTGCTCCTATCGAAAAGACCATC TCCAAGGCCAAGGGCCAGCCTAGGGAACCCCAGGTTTACACCTTGCCTCCATCTCG GGACGAGCTGACCAAGAACCAGGTGTCCCTGACCTGTCTCGTGAAGGGCTTCTACC CCTCCGATATCGCCGTGGAATGGGAGTCTAATGGCCAGCCAGAGAACAACTACAAG ACAACCCCTCCTGTGCTGGACTCCGACGGCTCATTCTTTCTGTACTCCAAGCTGACA GTGGACAAGTCCAGATGGCAGCAGGGCAACGTGTTCTCCTGCAGCGTGATGCACGA GGCCCTGCACAATCACTACACACAGAAGTCCCTGTCTCTGTCCCCTGGCAAG |

[Table 9-19]

| | | | |
|---|---|---|---|
| Table 9 (continued) | | | |
| 33 | IdeZ variant A-human Fc variant 18 | Synthetic construct | DNA | GACGACTACCAGAGAAATGCCGCCGAGGTGTACGCCAAAGAGGTGCCACATCAGA TCACCTCCGTGTGGACCAAGGGCGTGACACCTCTGACACCCGAGCAGTTCCGGTAC AACAACGAGGATGTGATCCACGCTCCTTACCTGGCTCACCAAGGATGGTACGACAT CACCAAGGTGTTCGACGGCAAGGACAACCTGCTGTGTGGCGCTGCTACCGCCGGCA ATATGCTGCACTGGTGGTTCGACCAGAACAAAGAGGAAATCGAGGCCTACCTGAGC AAGCACCCCGAGAAGCAGAAGATCATCTTCAACAATCAAGAGCTGTTCGACCTGAA GGCCGCCATCGACACCAAGGACTCCCAGACCAACAGCCAGCTGTTCAACTACTTCC GCGACAAGGCCTTTCCTGGCCTGTCCGCTAGACAGCTGGGAGTGATGCCTGACCTG GTGCTGGACATGTTCATCAACGGCTACTACCTGAACGTGTTCAAGACCCAGTCCACC GACGTGAACCGGCCTTACCAGGACAAGGATAAGAGAGGCGGCATCTTCGACGCCGT GTTCACCAGAGGCGATCAGACCACACTGCTGACCGCCAGACACGATCTGAAGAACA AGGGCCTGAACGACATCTCCACCATCATCAAGCAAGAGCTGACCGAGGGCAGAGCC CTGGCTCTGTCTCACACCTATGCCAACGTGCGGATCTCCCACGTGATCAACCTGTGG GGCGCTGACTTTAACGCCGAGGGCAATCTGGAAGCCATCTACGTGACCGACTCTGA CGCCGATGCCTCCATCGGCATGAAGAAATACTTCGTGGGCATCAACGCCCACGGCC ATGTGGCCATCTCTGCCAAGAAGATCGAGGGCGAGAATATCGGCGCTCAGGTGCTG GGCCTGTTCACCCTGAGTTCCGGCAAGGATATCTGGCAGAAGCTGTCCGACAAGAC CCACACCTGTCACAAGTGCCCTGCTCCTAACGCCGAAGAGGTGTCCTCCGTGTTTCT GTTCCCTCCAAAGCCTAAGGACACCCTGATGGCCTCTCGGACCCCTGAAGTGACAT GCGTGGTGGTGGATGTGTCCCACGAGGACCCAGAAGTGAAGTTCAATTGGTACGTG GACGGCGTGGAAGTGCACAACGCCAAGACCAAGCCTAGAGAGCAGgccAACTCCAC CTACAGAGTGGTGTCCGTGCTGACCGTGCTGCACCAGGATTGGCTGAACGGCAAAG AGTACAAGTGCAAGGTGTCCAACAAGGCCCTGCCTGCTCCAATCGAAAAGACCATC TCCAAGGCCAAGGGCCAGCCTAGGGAACCCCAGGTTTACACCTTGCCTCCATCTCG GGACGAGCTGACCAAGAACCAGGTGTCCCTGACCTGTCTCGTGAAGGGCTTCTACC CCTCCGATATCGCCGTGGAATGGGAGTCTAATGGCCAGCCAGAGAACAACTACAAG ACAACCCCTCCTGTGCTGGACTCCGACGGCTCATTCTTTCTGTACTCCAAGCTGACA GTGGACAAGTCCAGATGGCAGCAGGGCAACGTGTTCTCCTGCAGCGTGATGCACGA GGCCCTGCACAATCACTACACACAGAAGTCCCTGTCTCTGTCCCCTGGCAAG |

EP 4 772 624 A1

[Table 9-20]

Table 9 (continued)

| 34 | IdeZ variant A-human Fc variant 19_holes | Synthetic construct | DNA | GACGACTACCAGAGAAATGCCGCCGAGGTGTACGCCAAAGAGGTGCCACATCAGATCACCTCCGTGTGGACCAAGGGCGTGACACCTCTGACACCCGAGCAGTTCCGGTACAACAACGAGGATGTGATCCACGCTCCTTACCTGGCTCACCAAGGATGGTACGACATCACCAAGGTGTTCGACGGCAAGGACAACCTGCTGTGTGGCGCTGCTACCGCCGGCAATATGCTGCACTGGTGGTTCGACCAGAACAAAGAGGAAATCGAGGCCTACCTGAGCAAGCACCCCGAGAAGCAGAAGATCATCTTCAACAATCAAGAGCTGTTCGACCTGAAGGCCGCCATCGACACCAAGGACTCCCAGACCAACAGCCAGCTGTTCAACTACTTCCGCGACAAGGCCTTTCCTGGCCTGTCCGCTAGACAGCTGGGAGTGATGCCTGACCTGGTGCTGGACATGTTCATCAACGGCTACTACCTGAACGTGTTCAAGACCCAGTCCACCGACGTGAACCGGCCTTACCAGGACAAGGATAAGAGAGGCGGCATCTTCGACGCCGTGTTCACCAGAGGCGATCAGACCACACTGCTGACCGCCAGACACGATCTGAAGAACAAGGGCCTGAACGACATCTCCACCATCATCAAGCAAGAGCTGACCGAGGGCAGAGCCCTGGCTCTGTCTCACACCTATGCCAACGTGCGGATCTCCCACGTGATCAACCTGTGGGGCGCTGACTTTAACGCCGAGGGCAATCTGGAAGCCATCTACGTGACCGACTCTGACGCCGATGCCTCCATCGGCATGAAGAAATACTTCGTGGGCATCAACGCCCACGGCCATGTGGCCATCTCTGCCAAGAAGATCGAGGGCGAGAATATCGGCGCTCAGGTGCTGGGCCTGTTCACCCTGAGTTCCGGCAAGGATATCTGGCAGAAGCTGTCCGACAAGACCCACACCTGTCCTCCATGTCCTGCTCCAGAGGCTGAAGAGGTCCCCTCCGTGTTTCTGTTCCCTCCAAAGCCTAAGGACACCCTGATGGCCTCTCGGACCCCTGAAGTGACATGCGTGGTGGTGGATGTGTCCCACGAGGATCCCGAAGTGAAGTTCAATTGGTACGTGGACGGCGTGGAAGTGCACAACGCCAAGACCAAGCCTAGAGAGCAGTACAACTCCACCTACAGAGTGGTGTCCGTGCTGACCGTGCTGCACCAGGATTGGCTGAACGGCAAAGAGTACAAGTGCAAGGTGTCCAACAAGGCCCTGCCTGCTCCTATCGAAAAGACCATCTCCAAGGCCAAGGGCCAGCCTAGGGAACCCCAGGTTTACACCTTGCCACCTTCTCGGGACGAGCTGACCAAGAACCAGGTGTCCCTGTCTTGTGCCGTGAAGGGCTTCTACCCCTCCGATATCGCTGTGGAATGGGAGAGCAATGGCCAGCCTGAGAACAACTACAAGACAACCCCTCCTGTGCTGGACTCCGACGGCTCATTCTTCCTGGTGTCCAAGCTGACAGTGGACAAGTCCAGATGGCAGCAGGGCAACGTGTTCTCCTGCAGCGTGATGCACGAGGCCCTGCACAATCACTACACCCAGAAGTCTCTGTCTCTGTCCCCTGGCAAG |

EP 4 772 624 A1

[Table 9-21]

Table 9 (continued)

| 35 | human Fc variant 19_knobs | Synthetic construct | DNA | TGTCCTCCATGTCCTGCACCTGAGGCTGAAGAGGTGCCCTCCGTGTTTCTGTTCC CTCCAAAGCCTAAGGACACCCTGATGGCCTCTCGGACCCCTGAAGTGACATGCG TGGTGGTGGATGTGTCCCACGAGGATCCCGAAGTGAAGTTCAATTGGTACGTGG ACGGCGTGGAAGTGCACAACGCCAAGACCAAGCCTAGAGAGCAGTACAACTCC ACCTACAGAGTGGTGTCCGTGCTGACCGTGCTGCACCAGGATTGGCTGAACGGC AAAGAGTACAAGTGCAAGGTGTCCAACAAGGCCCTGCCTGCTCCTATCGAAAA GACCATCTCCAAGGCCAAGGGCCAGCCTAGGGAACCCCAGGTTTACACCTTGCC TCCATCTCGGGACGAGCTGACCAAGAACCAGGTGTCCCTGTGGTGTCTGGTCAA GGGCTTCTACCCCTCCGATATCGCCGTGGAATGGGAGTCTAATGGCCAGCCTGA GAACAACTACAAGACCACACCTCCTGTGCTGGACTCCGACGGCTCATTCTTTCT GTACTCCAAGCTGACAGTGGACAAGTCCAGATGGCAGCAGGGCAACGTGTTCT CCTGCAGCGTGATGCACGAGGCCCTGCACAATCACTACACCCAGAAGTCCCTGT CTCTGTCCCCTGGCAAG |

Although some embodiments of the present invention are described above, these embodiments are presented as examples and do not intend to limit the scope of the invention. These novel embodiments may be carried out in other various forms, and various omissions, replacements, and changes or modifications can be made therein without departing from the gist of the invention. These embodiments or their modifications are included in the scope and the gist of the invention and also included in the scope of aspects of the invention described in claims and a scope equivalent thereto.

Industrial Applicability

The IdeZ variant of the present invention and a polypeptide and the like comprising the IdeZ variant have favorable protease activity even when produced using mammalian cells, and are useful for the pretreatment of gene therapy using AAV and the prevention or treatment of rejection in transplantation, autoimmune diseases, and the like.

**Claims**

1. An IdeZ variant having IgG cysteine protease activity, the variant

   (a) comprising a sequence 90% or more identical to an amino acid sequence from positions 35 to 349 of the amino acid sequence represented by SEQ ID NO: 1; and
   (b) having unglycosylated amino acids at a position corresponding to position 118 and a position corresponding to position 267 of the amino acid sequence represented by SEQ ID NO: 1.

2. The IdeZ variant according to claim 1, wherein the IdeZ variant comprises the following amino acid variations (b') and (b"):

   (b')

   (i) an amino acid at position corresponding to position 118 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;
   (ii) an amino acid at position corresponding to position 119 of the amino acid sequence represented by SEQ ID NO: 1 is substituted by proline; and/or
   (iii) an amino acid at position corresponding to position 120 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane; and

   (b")

   (i) an amino acid at position corresponding to position 267 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;
   (ii) an amino acid at position corresponding to position 268 of the amino acid sequence represented by SEQ ID NO: 1 is substituted by proline; and/or
   (iii) an amino acid at position corresponding to position 269 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane.

3. The IdeZ variant according to claim 2, wherein in the IdeZ variant, an amino acid at position corresponding to position 120 of the amino acid sequence represented by SEQ ID NO: 1 is substituted by glutamic acid, and an amino acid at position corresponding to position 269 of the amino acid sequence represented by SEQ ID NO: 1 is substituted by arginine.

4. The IdeZ variant according to any one of claims 1 to 3, wherein the IdeZ variant has (c) an unglycosylated amino acid at position corresponding to position 171 of the amino acid sequence represented by SEQ ID NO: 1, and/or (d) an unglycosylated amino acid at position corresponding to position 298 of the amino acid sequence represented by SEQ ID NO: 1.

5. The IdeZ variant according to claim 4, wherein the IdeZ variant comprises the following amino acid variations (c') and/or (d'):

(c')

(i) an amino acid at position corresponding to position 171 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;
(ii) an amino acid at position corresponding to position 172 of the amino acid sequence represented by SEQ ID NO: 1 is substituted by proline; and/or
(iii) an amino acid at position corresponding to position 173 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane;

(d')

(i) an amino acid at position corresponding to position 298 of the amino acid sequence represented by SEQ ID NO: 1 is substituted by an amino acid selected from the group consisting of glycine, alanine, serine, proline, valine, threonine, cysteine, isoleucine, leucine, aspartic acid, glutamine, lysine, glutamic acid, methionine, histidine, phenylalanine, arginine, tyrosine, and tryptophane;
(ii) an amino acid at position corresponding to position 299 of the amino acid sequence represented by SEQ ID NO: 1 is substituted by proline; and/or
(iii) an amino acid at position corresponding to position 300 of the amino acid sequence represented by SEQ ID NO: 1 is substituted by an amino acid selected from the group consisting of glycine, alanine, proline, valine, cysteine, isoleucine, leucine, asparagine, aspartic acid, glutamine, lysine, glutamic acid, methionine, histidine, phenylalanine, arginine, tyrosine, and tryptophane.

6. The IdeZ variant according to claim 5, wherein in the IdeZ variant, an amino acid at position corresponding to position 171 of the amino acid sequence represented by SEQ ID NO: 1 is substituted by glycine, and/or an amino acid at position corresponding to position 298 of the amino acid sequence represented by SEQ ID NO: 1 is substituted by aspartic acid.

7. The IdeZ variant according to claim 6, wherein

in the IdeZ variant,
an amino acid at position corresponding to position 120 of the amino acid sequence represented by SEQ ID NO: 1 is substituted by glutamic acid,
an amino acid at position corresponding to position 171 of the amino acid sequence represented by SEQ ID NO: 1 is substituted by glycine,
an amino acid at position corresponding to position 269 of the amino acid sequence represented by SEQ ID NO: 1 is substituted by arginine, and
an amino acid at position corresponding to position 298 of the amino acid sequence represented by SEQ ID NO: 1 is substituted by aspartic acid.

8. The IdeZ variant according to claim 7, wherein the IdeZ variant comprises an amino acid sequence 90% or more identical to the amino acid sequence represented by SEQ ID NO: 5.

9. A polypeptide comprising the IdeZ variant according to any one of claims 1 to 8.

10. The polypeptide according to claim 9, wherein the polypeptide is a fusion protein of the IdeZ variant and an Fc region.

11. The polypeptide according to claim 10, wherein the Fc region is derived from human IgG.

**12.** The polypeptide according to claim 11, wherein the Fc region is derived from human Igγ1, human Igy2, human Igy3, or human Igy4.

**13.** The polypeptide according to any one of claims 10 to 12, wherein

in the Fc region,
amino acid residue 236 is substituted by glutamic acid, and
amino acid residue 237 is substituted by valine,

wherein the amino acid residue numbers are amino acid positions according to the EU index.

**14.** The polypeptide according to claim 13, wherein

in the Fc region, in addition,
amino acid residue 234 is substituted by alanine, and
amino acid residue 235 is substituted by glutamic acid.

**15.** The polypeptide according to claim 14, wherein

in the Fc region, in addition,
amino acid residue 227 is substituted by histidine,
amino acid residue 228 is substituted by lysine,
amino acid residue 233 is substituted by asparagine, and/or
amino acid residue 238 is substituted by serine.

**16.** The polypeptide according to claim 14 or 15, wherein

in the Fc region, in addition,
amino acid residue 253 is substituted by alanine,
amino acid residue 293 or 294 is deleted, and/or
amino acid residue 296 is substituted by alanine.

**17.** The polypeptide according to claim 16, wherein the Fc region is selected from the following Fc regions (1) and (2):

(1)

an Fc region in which
amino acid residue 234 is substituted by alanine,
amino acid residue 235 is substituted by glutamic acid,
amino acid residue 236 is substituted by glutamic acid,
amino acid residue 237 is substituted by valine,
amino acid residue 253 is substituted by alanine, and
amino acid residue 293 or 294 is deleted; and

(2)

an Fc region in which
amino acid residue 227 is substituted by histidine,
amino acid residue 228 is substituted by lysine,
amino acid residue 233 is substituted by asparagine,
amino acid residue 234 is substituted by alanine,
amino acid residue 235 is substituted by glutamic acid,
amino acid residue 236 is substituted by glutamic acid,
amino acid residue 237 is substituted by valine,
amino acid residue 238 is substituted by serine,
amino acid residue 253 is substituted by alanine, and
amino acid residue 293 or 294 is deleted.

18. The polypeptide according to claim 17, wherein the Fc region comprises an amino acid sequence 90% or more identical to a sequence consisting of amino acids at positions 316 to 541 in the amino acid sequence represented by SEQ ID NO: 16, a sequence consisting of amino acids at positions 316 to 541 in the amino acid sequence represented by SEQ ID NO: 17, a sequence consisting of amino acids at positions 316 to 541 in the amino acid sequence represented by SEQ ID NO: 21, or a sequence consisting of amino acids at positions 316 to 541 in the amino acid sequence represented by SEQ ID NO: 22.

19. The polypeptide according to any one of claims 10 to 18, wherein the fusion protein is a fusion protein of the IdeZ variant linked to the N terminus of the Fc region, and two such fusion proteins form a dimer.

20. The polypeptide according to any one of claims 10 to 18, wherein the polypeptide comprises a fusion protein of the IdeZ variant linked to the N terminus of a first Fc region, and a second Fc region without the IdeZ variant linked thereto.

21. The polypeptide according to claim 20, wherein

the first Fc region and the second Fc region comprise knobs-into-holes mutations, and
the knobs-into-holes mutations are any of the following mutations (1) and (2):

(1)

in the first Fc region, amino acid residue 366 is substituted by tryptophane, and
in the second Fc region, amino acid residue 366 is substituted by serine, amino acid residue 368 is substituted by alanine, and amino acid residue 407 is substituted by valine; and

(2)

in the first Fc region, amino acid residue 366 is substituted by serine, amino acid residue 368 is substituted by alanine, and amino acid residue 407 is substituted by valine, and
in the second Fc region, amino acid residue 366 is substituted by tryptophane.

22. The polypeptide according to any one of claims 19 to 21, wherein the Fc region or the first Fc region and the second Fc region are sialylated.

23. A polynucleotide encoding the polypeptide according to any one of claims 9 to 22.

24. An expression vector comprising the polynucleotide according to claim 23.

25. A host cell transformed with the expression vector according to claim 24.

26. A method for producing a polypeptide comprising an IdeZ variant, the method comprising the step of culturing the host cell according to claim 25.

27. The method according to claim 26, further comprising the step of adding or expressing $\beta$-galactoside $\alpha$-2,6-sialyltransferase 1 (ST6GAL1).

28. An Fc region variant wherein

amino acid residue 236 is substituted by glutamic acid, and
amino acid residue 237 is substituted by valine,

wherein the amino acid residue numbers are amino acid positions according to the EU index.

29. The Fc region variant according to claim 28, wherein, in addition,

amino acid residue 234 is substituted by alanine, and
amino acid residue 235 is substituted by glutamic acid.

30. The Fc region variant according to claim 29, further comprising the following amino acid variation(s):

amino acid residue 227 is substituted by histidine,
amino acid residue 228 is substituted by lysine,
amino acid residue 233 is substituted by asparagine, and/or
amino acid residue 238 is substituted by serine.

31. The Fc region variant according to claim 29 or 30, further comprising the following amino acid variation(s):

amino acid residue 253 is substituted by alanine,
amino acid residue 293 or 294 is deleted, and/or
amino acid residue 296 is substituted by alanine.

32. The Fc region variant according to claim 31, wherein the Fc region variant is selected from the following Fc region variants (1) and (2):

(1)

an Fc region variant in which
amino acid residue 234 is substituted by alanine,
amino acid residue 235 is substituted by glutamic acid,
amino acid residue 236 is substituted by glutamic acid,
amino acid residue 237 is substituted by valine,
amino acid residue 253 is substituted by alanine, and
amino acid residue 293 or 294 is deleted; and

(2)

an Fc region variant in which
amino acid residue 227 is substituted by histidine,
amino acid residue 228 is substituted by lysine,
amino acid residue 233 is substituted by asparagine,
amino acid residue 234 is substituted by alanine,
amino acid residue 235 is substituted by glutamic acid,
amino acid residue 236 is substituted by glutamic acid,
amino acid residue 237 is substituted by valine,
amino acid residue 238 is substituted by serine,
amino acid residue 253 is substituted by alanine, and
amino acid residue 293 or 294 is deleted.

33. The Fc region variant according to any one of claims 28 to 32, wherein the Fc region is derived from human IgG.

34. The Fc region variant according to claim 33, wherein the Fc region is derived from human Igγ1, human Igγ2, human Igγ3, or human Igγ4.

35. A polypeptide comprising the Fc region variant according to any one of claims 28 to 34 which is not cleaved with IgG cysteine protease.

36. A fusion protein of IgG cysteine protease and the Fc region variant according to any one of claims 28 to 34.

37. The fusion protein according to claim 36, wherein the IgG cysteine protease is IdeS, IdeZ, IdeZ2, IdeE, IdeE2, IdeP, IdeSORK, or IgdE, or a variant thereof.

38. The fusion protein according to claim 37, wherein the fusion protein comprises an amino acid sequence 90% or more identical to a sequence consisting of amino acids at positions 316 to 541 in the amino acid sequence represented by SEQ ID NO: 16, a sequence consisting of amino acids at positions 316 to 541 in the amino acid sequence represented by SEQ ID NO: 17, a sequence consisting of amino acids at positions 316 to 541 in the amino acid sequence represented by SEQ ID NO: 21, or a sequence consisting of amino acids at positions 316 to 541 in the amino acid sequence represented by SEQ ID NO: 22.

Fig. 1

Non-reducing

Reducing

Fig. 2

Fig. 3

Non-reducing

Reducing

Fig. 4

Fig. 5

**Reducing condition**                    **Non-reducing condition**

Fig. 6

**Reducing condition**                    **Non-reducing condition**

Fig. 7

Reducing condition                    Non-reducing condition

Fig. 8

## INTERNATIONAL SEARCH REPORT

| International application No. |
|---|
| **PCT/JP2024/030890** |

| | |
|---|---|
| **A. CLASSIFICATION OF SUBJECT MATTER** | |

*C12N 9/52*(2006.01)i; *C07K 16/18*(2006.01)i; *C07K 19/00*(2006.01)i; *C12N 1/15*(2006.01)i; *C12N 1/19*(2006.01)i; *C12N 1/21*(2006.01)i; *C12N 5/10*(2006.01)i; *C12N 9/10*(2006.01)i; *C12N 15/62*(2006.01)i; *C12N 15/63*(2006.01)i
FI:   C12N9/52 ZNA; C07K19/00; C07K16/18; C12N1/15; C12N1/19; C12N1/21; C12N5/10; C12N9/10; C12N15/62 Z; C12N15/63 Z

According to International Patent Classification (IPC) or to both national classification and IPC

| | |
|---|---|
| **B. FIELDS SEARCHED** | |

Minimum documentation searched (classification system followed by classification symbols)

C12N9/52; C07K16/18; C07K19/00; C12N1/15; C12N1/19; C12N1/21; C12N5/10; C12N9/10; C12N15/62; C12N15/63

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq

| | |
|---|---|
| **C. DOCUMENTS CONSIDERED TO BE RELEVANT** | |

| Category* | Citation of document, with indication, where appropriate, of the relevant passages | Relevant to claim No. |
|---|---|---|
| X | WO 2021/233911 A1 (HANSA BIOPHARMA AB) 25 November 2021 (2021-11-25) claims, examples, table C | 1-9, 23-27 |
| Y | | 10-27, 36-38 |
| A | | 28-35 |
| X | WO 2022/057942 A1 (SHANGHAI BAOPHARMACEUTICALS CO., LTD.) 24 March 2022 (2022-03-24) claim 1-3, pp. 3, 11, 22 | 1-12, 23-27 |
| Y | | 13-27, 36-38 |
| A | | 28-35 |

☑ Further documents are listed in the continuation of Box C.       ☑ See patent family annex.

| | | | |
|---|---|---|---|
| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **28 October 2024** | **12 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

International application No.

**PCT/JP2024/030890**

**C.    DOCUMENTS CONSIDERED TO BE RELEVANT**

| Category* | Citation of document, with indication, where appropriate, of the relevant passages | Relevant to claim No. |
|---|---|---|
| X | JP 2007-525443 A (XENCOR, INC.) 06 September 2007 (2007-09-06) claims | 28-35 |
| Y | claims, paragraph [0008] | 10-27, 36-38 |
| A | | 1-9 |

Form PCT/ISA/210 (second sheet) (July 2022)

**INTERNATIONAL SEARCH REPORT**

| International application No. |
| --- |
| **PCT/JP2024/030890** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

# EP 4 772 624 A1

## INTERNATIONAL SEARCH REPORT
### Information on patent family members

International application No.

**PCT/JP2024/030890**

| Patent document cited in search report | | | Publication date (day/month/year) | Patent family member(s) | | | Publication date (day/month/year) |
|---|---|---|---|---|---|---|---|
| WO | 2021/233911 | A1 | 25 November 2021 | JP | 2023-526849 | A | |
| | | | | EP | 4153737 | A1 | |
| WO | 2022/057942 | A1 | 24 March 2022 | JP | 2023-541698 | A | |
| | | | | claims 1-3, paragraphs [0014], [0018], [0020], [0081] | | | |
| | | | | EP | 4215208 | A1 | |
| | | | | US | 2023/0374083 | A1 | |
| JP | 2007-525443 | A | 06 September 2007 | WO | 2004/099249 | A2 | |
| | | | | claims, paragraph [0008] | | | |
| | | | | US | 2004/0132101 | A1 | |
| | | | | EP | 1620467 | A2 | |

Form PCT/ISA/210 (patent family annex) (July 2022)

## REFERENCES CITED IN THE DESCRIPTION

### Patent documents cited in the description

- WO 2020159970 A
- WO 2021233911 A
- WO 9007861 A
- WO 2006131347 A
- WO 2016012285 A
- WO 1998050431 A
- WO 2013094723 A
- WO 2016016586 A

### Non-patent literature cited in the description

- *The 1st Academic Conference in Commemoration of the Establishment of the Antibody Society of Japan*, November 2022, 74
- *Drugs*, October 2020, vol. 80, 1859-1864
- *Am J Transplant*, November 2018, vol. 18 (11), 2752-2762
- *Nucleic Acids Res.*, 2015, vol. 43, W580-W584
- *Biochem. J.*, 1981, vol. 195, 639-644
- *Biochemistry*, 1999, vol. 38, 13589-13591
- *Biochem. J.*, 2001, vol. 355, 245-248
- *Biochem. J.*, 1997, vol. 323, 415-419
- *Vaccines*, 2021, vol. 9 (7), 724
- *Science*, 1959, vol. 130, 432-437
- *Virol*, 1959, vol. 8, 396
- *J. Am. Med. Assoc.*, 1967, vol. 199, 519
- *Exp. Biol. Med.*, 1950, vol. 73, 1-8
- *Proc. Natl. Acad. Sci. USA.*, 1985, vol. 82, 8404
- Molecular Cloning. Cold Spring Harbor Laboratory, vol. 3
- *Proc. Natl. Acad. Sci. USA.*, 1980, vol. 77, 4505
- *Protein Eng.*, 1987, vol. 1, 499-505
- *J. Immunol.*, 2019, vol. 202, 1582-1594
- *Intl. Immunol.*, 2006, vol. 18, 1759-1769